# EUROPEAN PATENT APPLICATION

(11) **EP 4 438 622 A1**
(43) Date of publication of application: **02.10.2024**
(21) Application number: 23165591.1
(22) Date of filing: 30.03.2023
(51) Int. Cl.: C07K 14/78, C12N 15/01, C12N 15/09, C12N 15/90, C12N 5/071

(54) **CHO CELLS WITH OPTIMIZED ECM PROFILE**

(71) Applicant: Sartorius Stedim Cellca GmbH, 89081 Ulm (DE)
(72) Inventor: Zehe, Christoph, 89081 Ulm (DE); Leroux, Ann-Cathrin, 89081 Ulm (DE); Rattay, Merle, 89081 Ulm (DE); Reif, Oscar-Werner, 89081 Ulm (DE)
(74) Representative: Eisenführ Speiser

(57) **Abstract**

The present invention relates to a CHO cell with a modified extracellular matrix (ECM) profile and to methods for identifying such profiles. The present invention also relates to a method for the production of at least one modified CHO cell according to the present invention. Furthermore, the present invention also relates to method for the production of at least one recombinant protein of interest. Moreover, the present invention relates to the use of at least one modified CHO cell according to the present invention for the production of at least one pharmaceutical drug. Additionally, the present invention relates to a kit comprising at least one modified CHO cell according to the present invention.

## Description

### Technical Field

The present invention relates to a CHO cell with a modified extracellular matrix (ECM) profile and to methods for identifying such profiles. The present invention also relates to a method for the production of at least one modified CHO cell according to the present invention. Furthermore, the present invention also relates to method for the production of at least one recombinant protein of interest. Moreover, the present invention relates to the use of at least one modified CHO cell according to the present invention for the production of at least one pharmaceutical drug. Additionally, the present invention relates to a kit comprising at least one modified CHO cell according to the present invention.

### Background of the invention

The manufacturing of biotherapeutic agents, including antibodies and other therapeutic drugs, represents an important pillar of drug manufacturing. Typically, biotherapeutic agents, which are also referred to as a biopharmaceuticals or biologics, are produced from cells (bacterial, yeast, plant or mammalian), which are referred to as host cells or host cell lines, using recombinant DNA technology. Chinese Hamster Ovary (CHO) cells are the workhorse for the manufacture of biopharmaceuticals. Indeed, CHO cells for manufacturing biopharmaceuticals account for over 70% of recombinant biopharmaceutical proteins, most of them being monoclonal antibodies (mAbs) and 35.5% of the total cumulative (1982-2014) biopharmaceutical product approvals (Lalonde 2017, see http://dx.doi.org/10.1016/j.jbiotec.2017.04.028; Walsh, 2014, Nat. Biotechnol. 2014; 32:992-1000). The dominant use of CHO as host cells is due to their capacity to correctly fold and post-translationally modify recombinant proteins and thus make them compatible for humans (Jaypal et al., 2007, Chemical Engineering Progress 103(10):40-47). In addition, CHO cells can produce a human-like glycosylation patterns when producing proteins, such as antibodies and other glycosylated molecules, which advantageously avoids undesired immune responses. Further, CHO cells are fast-growing cells capable of producing large amounts of recombinant proteins via their secretory pathway.

CHO thus represent a highly prominent system for the production of biopharmaceutical proteins (e.g., antibodies). The continuous growth of the therapeutic protein market in combination with the increasing pressure to reduce development and manufacturing costs results in a need for very efficient CHO expression cell lines, which are not only characterized by the need to achieve high purities of a biopharmaceutical product. Additionally, a high performance in production bioprocesses is simultaneously required. In particular, a high performance demands a fast proliferation, high cell concentrations, high specific productivities, high final titers, high viabilities and cell culture longevity. Although over the last decades performance increases have been achieved driven by the optimization of expression vectors, cell culture media and bioprocess technologies, further improvements are still needed. Especially, optimizations of CHO host cell lines using genetic engineering approaches hold a great potential and have not been systematically exploited yet. There is thus a high need in providing further modified CHO cell lines to cope with the increasing safety and yield focused needs of the industry. So far, much attention has been drawn to host cell protein (HCP) of CHO cells as part of the final product when thinking about optimization strategies. HCPs are process-related protein impurities, which are endogenously produced by the host organism or the host cell line during biotherapeutic manufacturing and production. For recombinant manufacturing of biotherapeutics in a cell, laborious and cost-intensive downstream purification processes are required in order to remove the majority (> 99%) HCPs from the final product. Typically, HCP screening on a protein level and removal during downstream processing (DSP) was thus in the focus of optimizing CHO cell based biopharmaceutical product production. Exactly the purification steps necessary to obtain the relevant levels of purity in the ultimate biotherapeutic product by removing HCPs, however, significantly contribute to the overall production costs. Purification inherently demands cost-intensive steps conducted under controlled manufacturing conditions that also lead to a decreased product yield.

Interestingly, so far no efforts have been made to study different approaches to optimize CHO cells as such from the very beginning in a targeted and concerted way by clustering the CHO cell transcriptome and proteome into necessary and dispensable proteins for high yield and high quality recombinant molecule production.

Current solutions to improve the bioprocess performance of CHO production cell lines are mainly focusing on singular optimization means like the modification of the content of individual contaminating HCPs, expression vectors, cell culture media and bioprocess technologies (cf. US 9,932,591 B2, US 2016/251411 A1, or Klingler F., et al. Biotechnol. Bioeng. 2021 Aug;118(8):3015-3028. doi: 10.1002/bit.27811). Solutions based on the systematic genetic engineering of CHO cells based on the preceding basic data generated via a profound transcriptome and/or proteome analysis obtained from different CHO cells expressing different recombinant molecules are not existing yet.

So far, systematic approaches are thus particularly missing that specifically define and evaluate the CHO cell transcriptome and protein profiles and patterns in a host cell in a first step to establish a sound data base for determining which of the identified proteins are expendable or even obstructive for the production of recombinant biotherapeutic drugs in CHO cells. Further, systematic, but usually labor intensive and cumbersome, comparisons of transcriptome and expression profile studies are missing for CHO cells to test and provide a generally applicable platform host cell, preferably a modified CHO cell, that has a significantly reduced expendable protein profile - and thus at the same time always a reduced HCP profile, as any expendable protein at the same time represents an HCP undesirable as co-purifying contaminant, whilst simultaneously maintaining or even improving the overall product yield and upstream and downstream processing parameters during recombinant biotherapeutic production in a mammalian cell, preferably a CHO cell.

Despite the acknowledged importance of CHO cells as hosts for recombinant protein production, so far no comprehensive profiles of CHO cell proteins, exists that was generated using representative sample sets derived from industry-relevant bioprocessing conditions as a for analyzing and generating targeted knock-out CHO banks with optimized characteristics.

Attempts going even further systematically studying and integrating the goals of reduced HCP content and, at the same time, an overall maintenance of bioprocess performance when optimizing CHO cells are currently completely missing. What is more, no comprehensive profiles proteins using representative sample sets derived from industry-relevant bioprocessing conditions have been generated, analyzed and used as a basis for knock-out studies so far. Further, the impact of single/multiple protein knock-outs on the performance of production cell lines (e.g., growth, productivity, titer, etc.) was not evaluated under industry-relevant bioprocessing conditions.

Consequently, there is still a great need to further optimize CHO cells for an improved performance (upstream and downstream) to make the cell as such more suitable for biopharmaceutical product production. It is thus an object of the present invention to define new ways of identifying CHO cell protein candidates in a targeted way that represent valuable targets for single and specifically multiple knock-outs by using large scale transcriptome and/or proteome big data studies. Further, it was an object to provide CHO cells with an improved bioprocess performance even if performing multiple knock-outs of CHO cell proteins.

### Summary of the Invention

The present invention tackles the problems described above by the generation of genetically optimized CHO cell lines which are characterized by the specific knock-out of ECM or ECM-related proteins, alone or in combination with a targeted knock-down or knock-out of at least one further HCP, resulting in a reduced metabolic burden and/or an improved bioprocess performance.

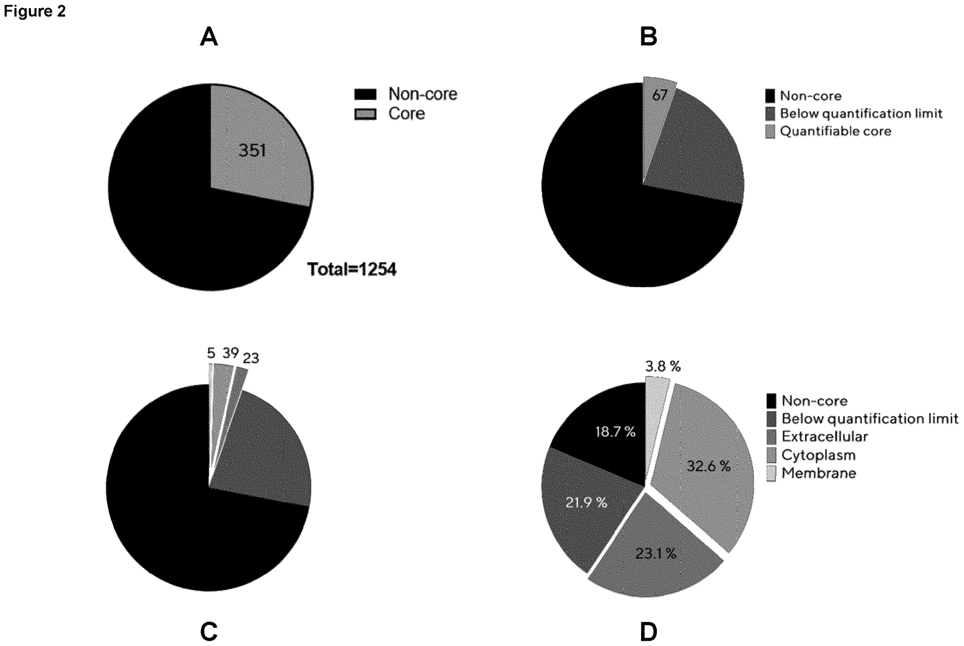

Originally being derived from the ovary of the Chinese hamster, CHO cells are epithelial cells by their very nature. Therefore, in their natural state they grow as adherent cells both *in situ* and in cell culture and are forming an extracellular matrix (ECM) by which they are surrounded. For their application in biopharmaceutical manufacturing processes, CHO cells have been adapted to non-adherent growth in suspension culture. Although no longer needed, many ECM proteins as well as proteins involved in the regulation, processing and assembly of ECM proteins are still expressed. The inventors found that this can have negative physiological impacts and pose an unnecessary metabolic burden on the cells by potentially restricting the bioprocess performance of the cell. Further, ECM and ECM-related proteins represent a significant and thus abundant amount of HCPs in a host cell the removal of which as byproduct of recombinant protein production is generally desirable (cf. **Fig. 2D**).

Surprisingly, a high throughput transcriptome based analysis provided data that allowed the identification of a specific ECM or ECM-related protein panel that represent interesting targets for knock-out studies, as these proteins as such are expendable for a CHO cell when cultivated in suspension culture so that CHO cells with a significantly reduced metabolic burden could be obtained based on the transcriptome profiling of ECM and ECM-related protein panels and profiles helping to optimized the genome of CHO cells towards even better recombinant molecule production strains.

In a first aspect, there is provided a CHO cell with a modified extracellular matrix (ECM) profile, characterized by a reduced load of at least one endogenous protein being an ECM protein or an ECM-related protein, preferably wherein said CHO cell comprises: at least one modification in at least one endogenous coding sequence coding for at least one endogenous ECM protein or endogenous ECM-related protein selected from the group summarized in Table 1, Table 4, Table 5, or Table 6, or of any homologue, orthologue, or paralogue thereof.

In one embodiment, there is provide a CHO cell comprising at least two or more modifications in two or more coding sequences coding for two or more endogenous ECM proteins and/or endogenous ECM-related proteins, wherein the at least one further modification of the at least two or more modifications is a modification of a sequence encoding an ECM protein or an ECM-related protein selected from the group summarized in Table 1, Table 4, Table 5, or Table 6, or any homologue, orthologue, or paralogue thereof, preferably wherein the at least one further modification of the at least two or more modifications is a modification of a sequence encoding an ECM protein or an ECM-related protein having an amino acid sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, respectively.

In another embodiment, there is provided a CHO cell, wherein said CHO cell comprises at least two or more modifications in two or more coding sequences coding for two or more endogenous ECM proteins and/or endogenous ECM-related proteins, wherein at least one of the at least two or more modifications is in a sequence encoding an ECM protein or an ECM-related protein being selected from the group consisting of Fibronectin, Basement membrane-specific heparan sulfate proteoglycan core protein, Peroxidasin, Biglycan, Galectin-3 binding protein, Laminin subunit beta-1, Calreticulin, Galectin, Glypican, or any homologue, orthologue, or paralogue thereof; more preferably wherein at least one of the at least two or more modifications is in a sequence encoding an ECM protein or an ECM-related protein having an amino acid sequence corresponding to SEQ ID NO: 2, 12, 29, 36, 38, 54, 55, 77, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: SEQ ID NO: 2, 12, 29, 36, 38, 54, 55, 77, respectively.

In yet another embodiment, there is provided a CHO cell, wherein the at least one further modification is a modification of a sequence encoding an ECM protein or an ECM-related protein selected from the group summarized in Table 1, Table 4, Table 5, or Table 6, or any homologue, orthologue, or paralogue thereof and/or wherein the at least one further modification is a modification of a sequence encoding an abundant core HCP (acHCP) and/or a difficult to remove HCP (drHCP) selected from the group summarized in Table 2 and Table 3 respectively, or any homologue, orthologue, or paralogue thereof.

In a further embodiment, there is provided a CHO cell, wherein said at least one modification leads to the reduced transcription and/or reduced functional expression of said endogenous ECM protein(s) and/or ECM-related protein(s) thereby lowering the total content of endogenous ECM proteins and/or endogenous ECM-related proteins.

In another embodiment, there is provided a CHO cell, wherein said CHO cell comprises three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, or twenty or more modifications in three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, or twenty or more coding sequences, respectively, wherein each of said modifications affects a protein being classified as an endogenous ECM protein or an endogenous ECM-related protein, preferably wherein at least one ECM protein or an endogenous ECM-related protein is independently selected from SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, respectively.

In another embodiment, there is provided a CHO cell, wherein said CHO comprises at least one recombinant gene encoding at least one recombinant protein of interest and/or encoding at least one recombinant RNA molecule of interest, preferably wherein said at least one recombinant protein of interest is a therapeutic molecule.

In yet another embodiment, there is provided a CHO cell, wherein said at least one modification is selected from the group consisting of at least one insertion, at least one deletions, and at least one substitution, including a base edit, or any combination thereof, preferably wherein said at least one modification is present in exon 1 of the respective endogenous coding sequence and/or wherein said at least one modification in said coding sequence is a frame-shift mutation or a point mutation, the point mutation resulting in a stop codon.

In one embodiment, there is provided a CHO cell, wherein said at least one modification in said at least one endogenous coding sequence allows for an improved bioprocess performance, wherein said improved bioprocess performance is characterized by an increased concentration of viable cells and/or an increased specific productivity and/or an increased final titer and/or an increased process duration in comparison to a wild-type cell not carrying the at least one modification in its genome.

In another embodiment, there is provided a cell, wherein said at least one modification in said endogenous coding sequence is a frame-shift mutation or a point mutation, the point mutation resulting in a stop codon.

In yet another embodiment, there is provided a CHO cell, wherein all alleles of said at least one endogenous coding sequence comprise at least one or more of said modification(s).

In a second aspect, there is provided method for the production of at least one modified CHO cell according to the first aspect above and the embodiments related to this aspect, the method comprising the steps: (i) providing at least one CHO cell comprising at least one endogenous target nucleic acid segment; (ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

In yet another aspect, there is provided a method for the production of at least one recombinant molecule, preferably at least one recombinant protein, of interest comprising the steps: (a) providing at least one modified CHO cell of the second aspect above, wherein the at least one modified CHO cell comprises at least one recombinant gene encoding at least one recombinant protein, DNA or RNA of interest; (b) culturing said at least one target cell in a culture medium such that at least one recombinant molecule of interest is transcribed and/or translated; (c) harvesting said at least one recombinant molecule of interest; (d) purifying and/or decontaminating said at least one recombinant molecule, preferably the at least one recombinant protein of interest.

In another embodiment, there is provided a use of at least one modified CHO cell according to the first aspect and the embodiments related thereto for the production of at least one pharmaceutical drug.

In another aspect, there is provided a kit comprising (a)at least one modified CHO cell according to the first aspect and the embodiments related thereto, the CHO cell or the kit comprising: (b.i) at least one nucleic acid molecule suitable for expressing at least one recombinant molecule of interest, preferably at least one recombinant protein of interest; optionally means for introducing the same into the CHO cell genome, or alternatively (b.ii) at least one nucleic acid molecule encoding at least one recombinant molecule of interest, preferably at least one recombinant protein of interest; optionally means for introducing the same into the CHO cell genome; or alternatively (b.iii)at least one nucleic acid molecule suitable for transcribing at least one recombinant RNA molecule of interest; optionally means for introducing the same into the CHO cell genome; or alternatively (b.iv) at least one nucleic acid molecule encoding at least one recombinant RNA molecule of interest; optionally means for introducing the same into the CHO cell genome; and optionally (c) culture medium suitable for the at least one modified CHO cell according to step (a) (i) to replicate the at least one nucleic acid molecule according to any of the steps (b.i), (b.ii), (b.iii), and (b.iv) endogenously or exogenously; and (ii.a) to express the at least one recombinant protein of interest according to step (b.i) and/or (b.ii), and/or to transcribe the at least one recombinant RNA molecule of interest according to step (b.iii) and/or (b.iv).

### Brief description of Figures

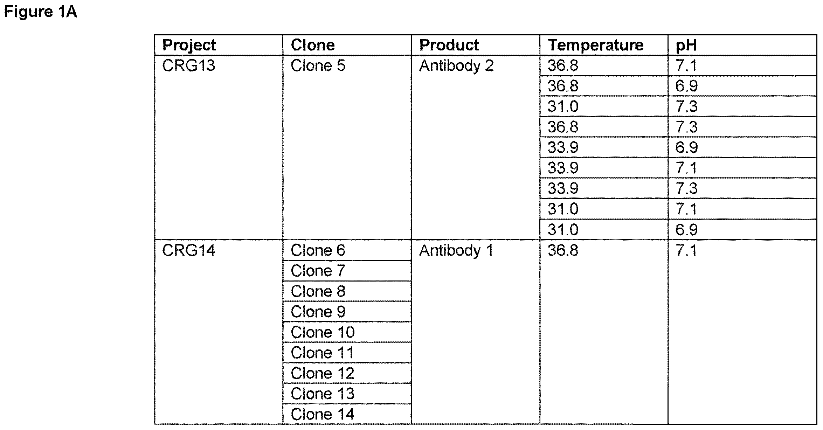

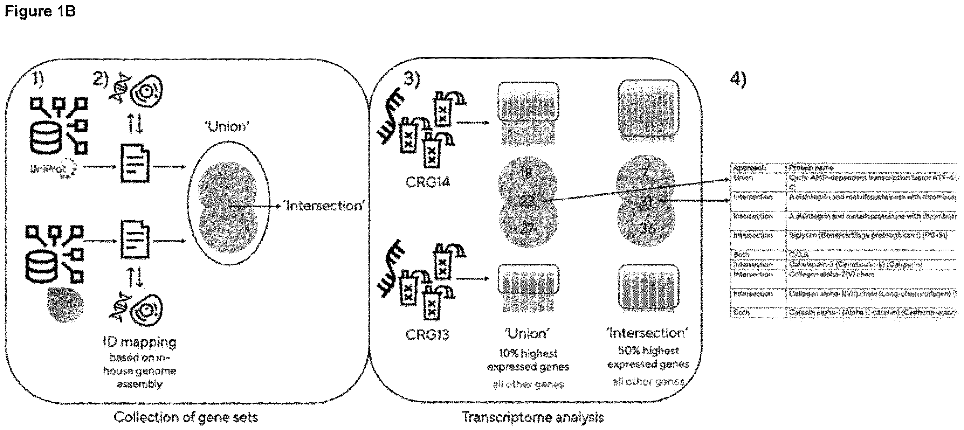

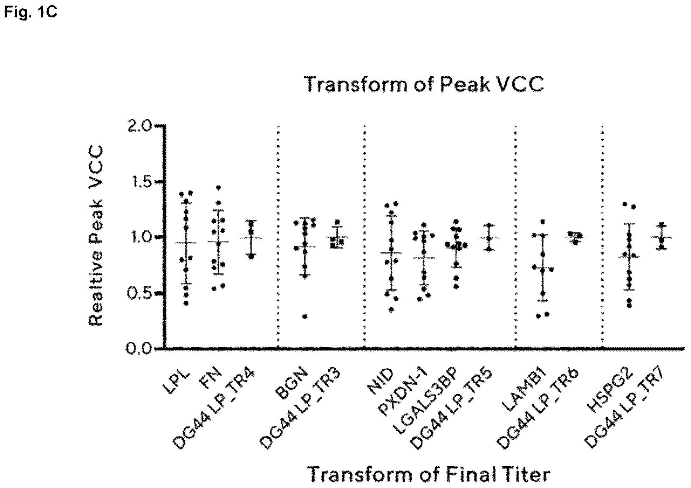

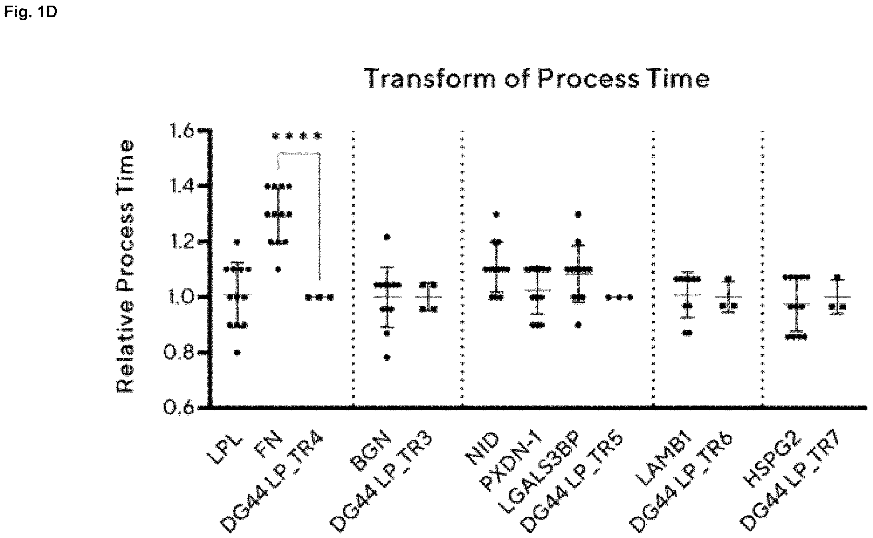

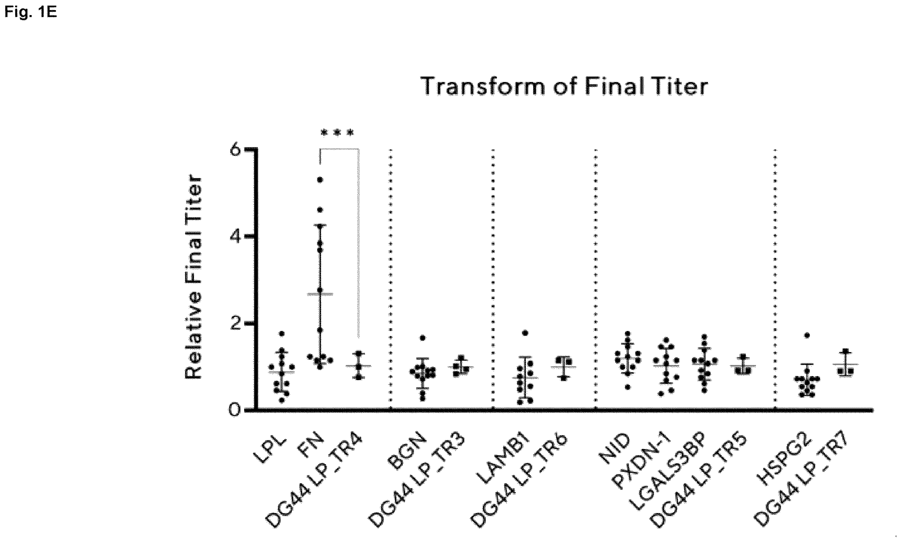

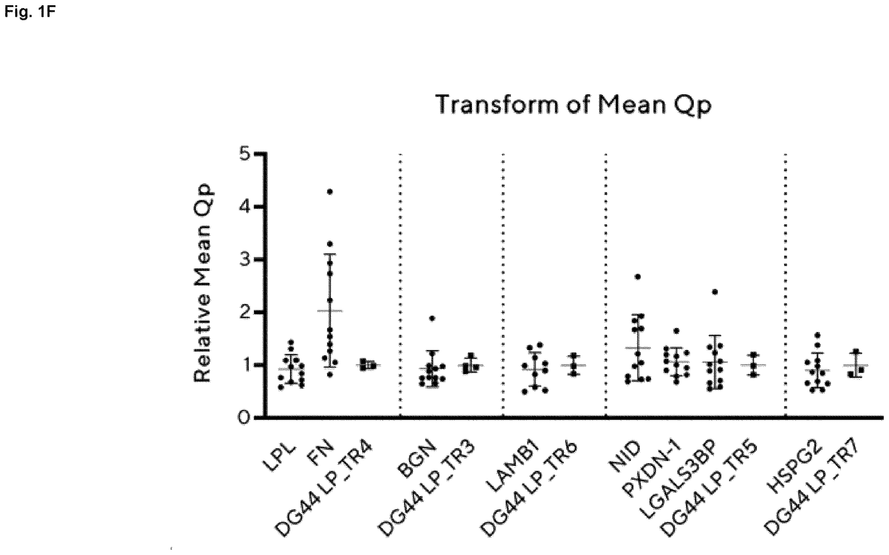

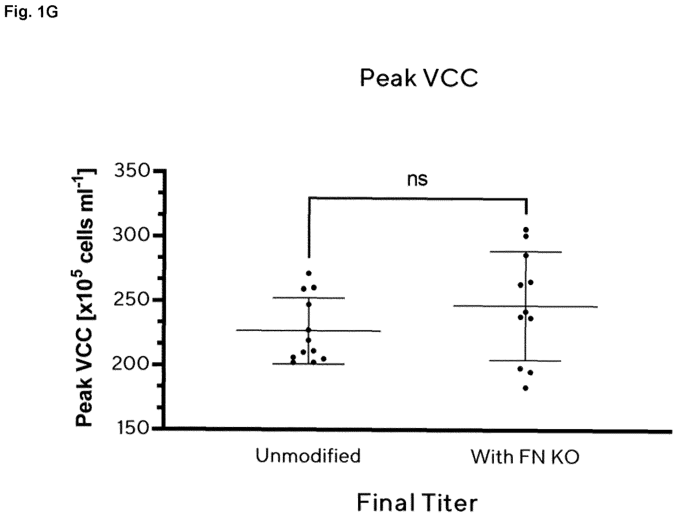

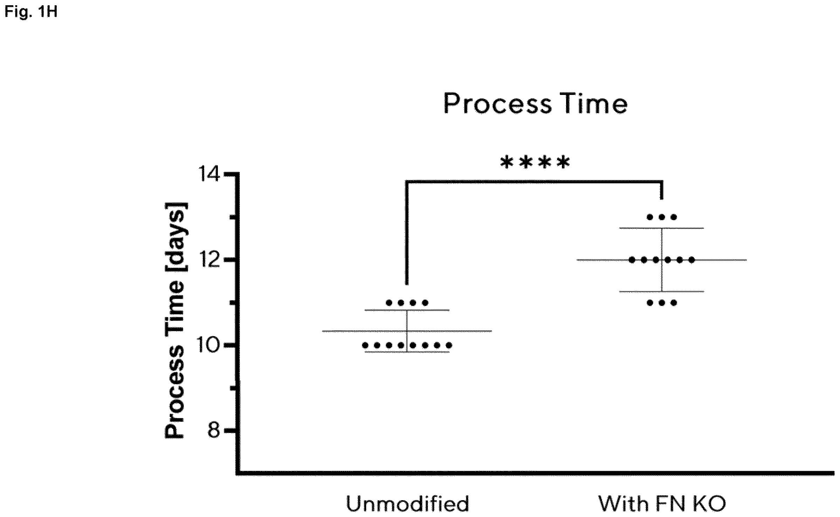

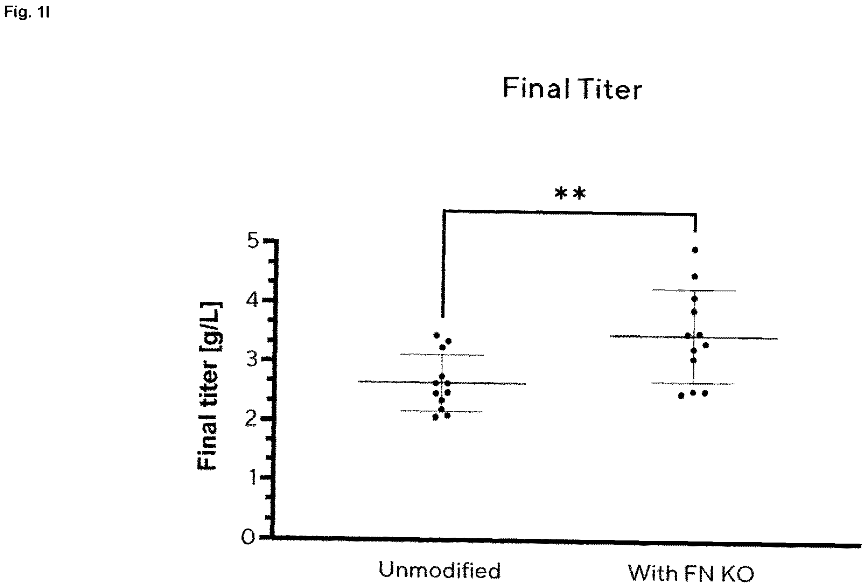

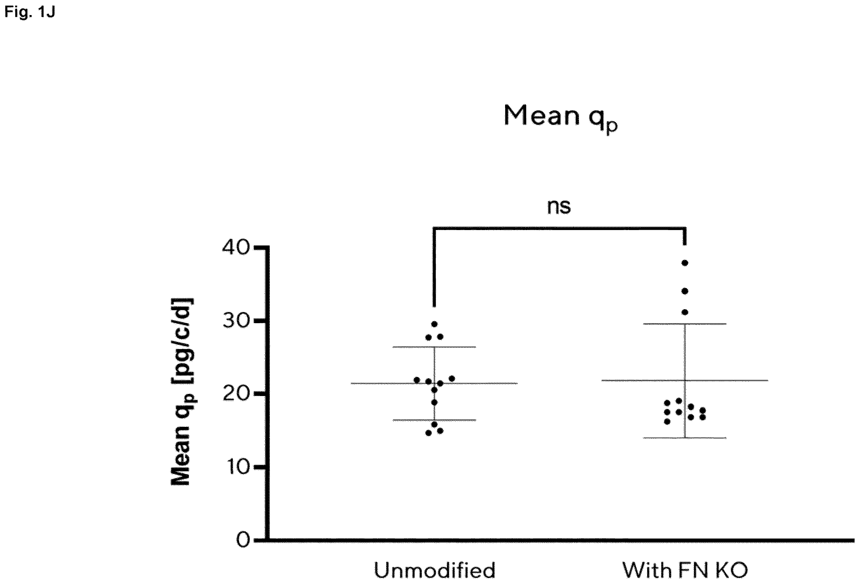

**Figure 1** **A** to **J** (**Fig. 1A** and **1J**) shows in **Fig. 1A**: an overview of production clones and bioprocess conditions used for the generation of transcriptome profiles used as basis for determining, calculating and comparing ECM and ECM-related protein subsets and profiles in an individual manner. **Fig. 1B**: Overview of bioinformatic analysis to identify relevant ECM or ECM-related knock-out targets. Gene sets were collected from two public databases (1)), identified in the in-house CHO genome assembly, and 'union' and 'intersection' gene sets defined (2)). In the transcriptome analysis, two RNA-Seq datasets (CRG13, CRG14; cf. **Fig. 1A**) were used to identify the core of most highly expressed ECM or ECM-related genes based on the 'union' and 'intersection' gene set (3)). Finally, the identified overlap of all datasets was merged into the final table of targets (4)). **Fig. 1C** shows the transform of Peak VCC (= viable cell concentration) by plotting the relative peak VCC (y-axis) determined for the generated clones detailed in the Examples (cf. **Example 1.3)** for the HCP targets as well as a respective CHO DG44 wild-type (WT) controls (TR4, TR3, TR5, TR6 and TR7, respectively - see also **Example 1.3** for further explanations). Fed-Batch endpoint is shown. **Fig. 1D**: Transform of process time. **Fig. 1E**: Transform of Final Titer on harvest day. **Fig. 1F**: Transform of mean Qp. A process ends once the viability falls below a pre-set threshold. Here, final viability, final titer [g/L] and final Qp [pg/c/d] are relevant. The cell specific productivity Qp is calculated using the cumulative integral of viable cell concentration (IVCC [×10⁵ cells*d/ml]) and titer for each day, respectively. Therefore, IVCC, process time and Qp describe the course of the bioprocess and its productivity. From the Qp, mean Qp is calculated, averaged over the time of cultivation. (Brown-Forysthe and Welch ANOVA test performed, Dunnett's test, alpha = 0.05)

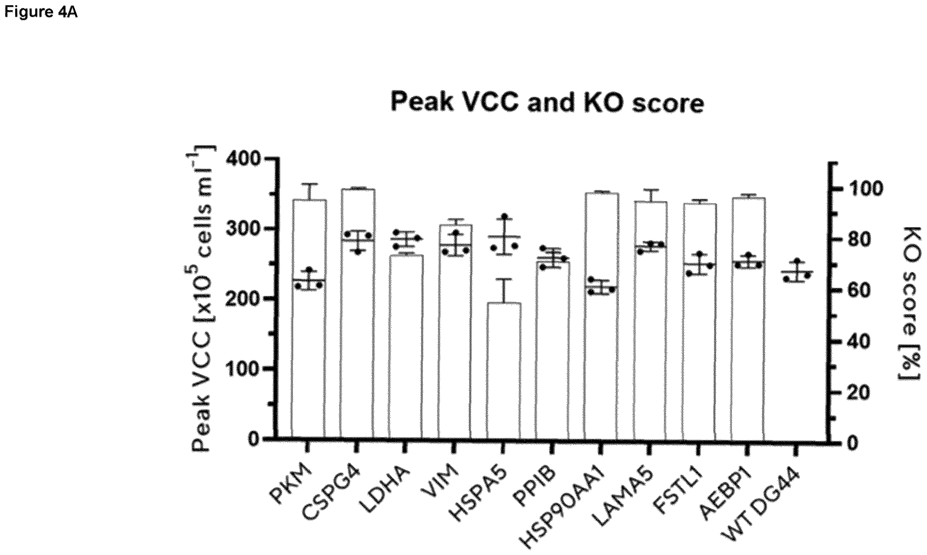

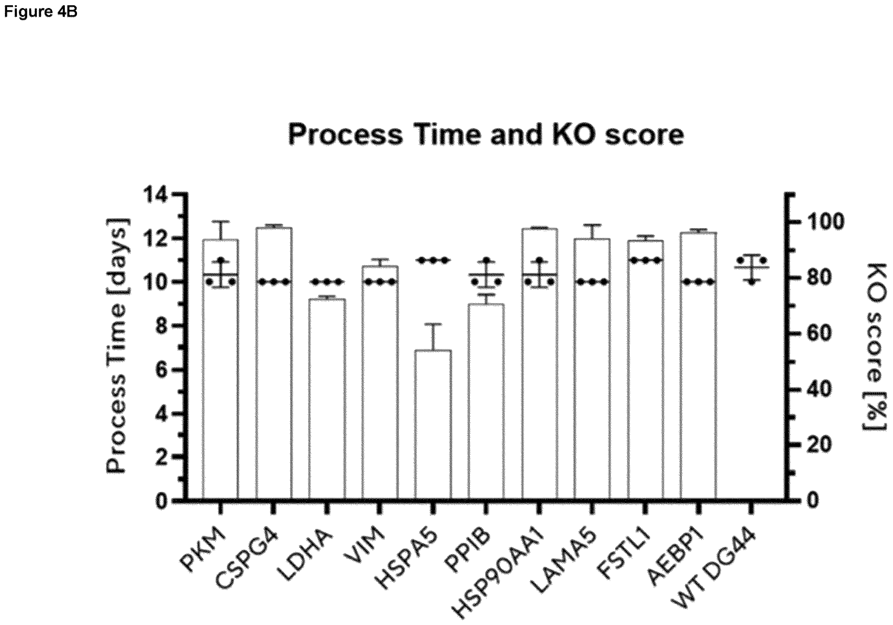

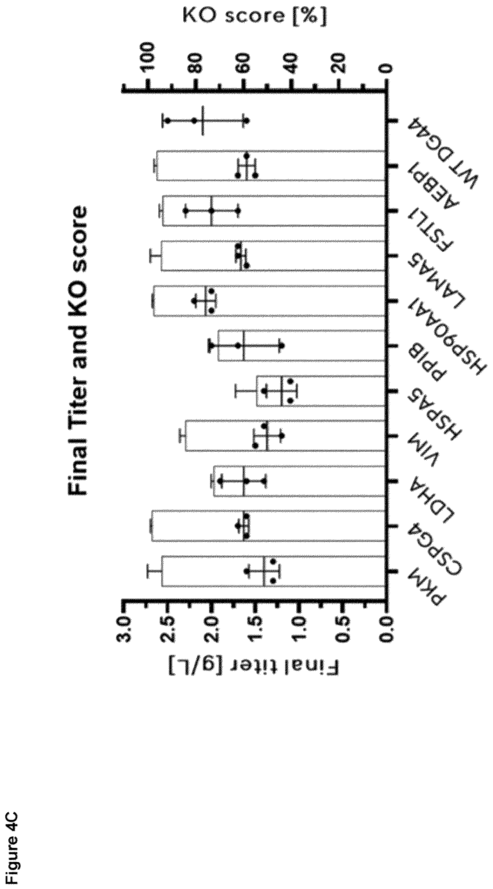

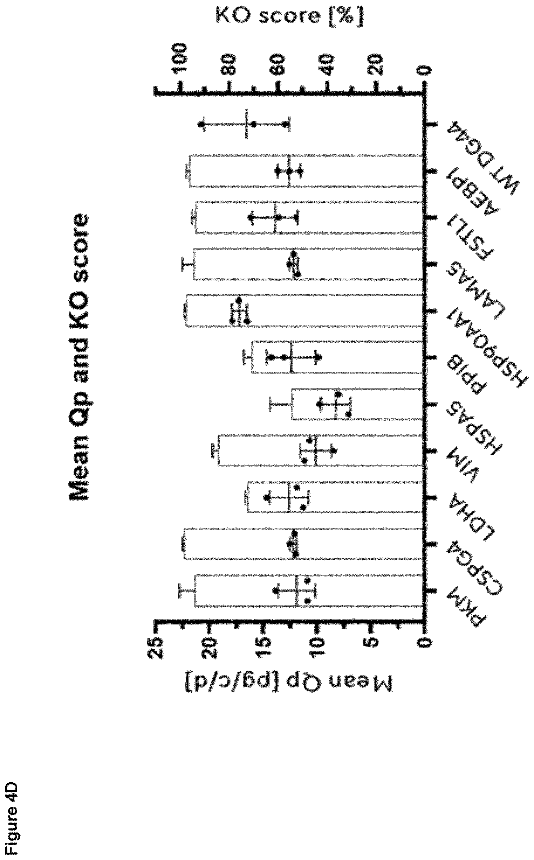

Figure 1G-J (Fig. 1G to J) shows the results of the experiments detailed in **Example 1.4** below, always measured for fibronectin as HCP knocked-out target (cf. also Example4 and Fig. 4 for additional candidates) as detailed in this Example and the wild-type (WT) control. **Fig. 1G**: shows the peak VCC (viable cell concentration in [×10⁵ cells*/ml]) and the KO score (in%). **Fig. 1H**: shows the process time (in days) and the KO score (in%). **Fig. 1I**: shows the final titer (in g/L) and the KO score (in%). **Fig. 1J**: shows final Qp [pg/c/d] and the KO score (in%). Ns= not significant.
   See **Example 1** and subsections (1.1 to 1.4) for further explanations.

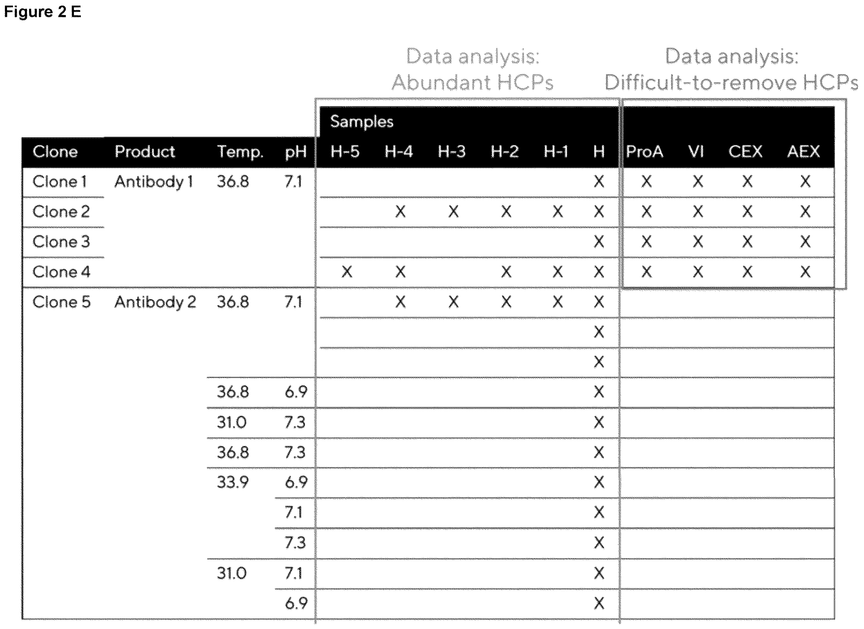

Figure 2A to E (Fig. 2A to E) gives a schematic overview on the identification process, classification and quantities of core HCPs. **Fig. 2A**: first round of analysis classifying the 1,254 HCPs tested into core and non-core. **Fig. 2B**: definition of the cluster of core HCPs quantifiable under the settings chosen characterized further (67). **Fig. 2C**: further characterization of the 67 quantifiable HCPs of **Fig. 2B**. Black = Non-core; grey = below LLOQ, 5 = 5 HCPs with membrane localization; 23 = 23 HCPs extracellular; 5 = 5 HCPs with membrane localization (in sum: 67 quantifiable core of **Fig. 2B**). **Fig. 2D**: shows the percentage of the 67 quantifiable core HCPs of **Fig. 2** **B** and **C** in relation to all 1,254 HCPs analyzed. **Figure 2E** shows an overview of samples measured by SWATH LC-MS and HCP profiles analyzed to define relevant abundant and difficult-to-remove HCPs as further described in **Example 2.** "H" means harvest. "H-5" thus means 5 days before harvest etc. pp. To obtain a comprehensive and comparable data set, several clones were tested producing different antibody constructs. ProA = Protein A column. VI = virus inactivation step. CEX = cation exchange chromatography. AEX = anion exchange chromatography. See also **Example 2** for further explanations.

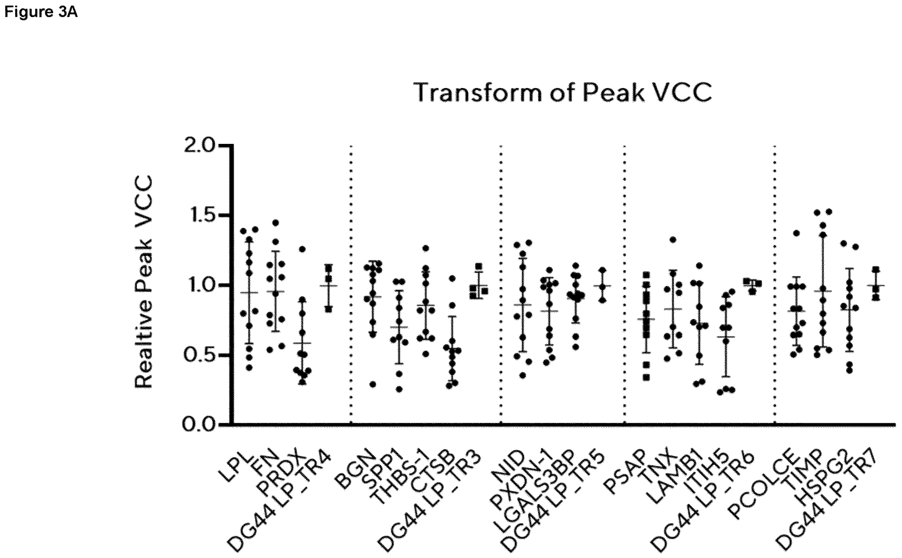

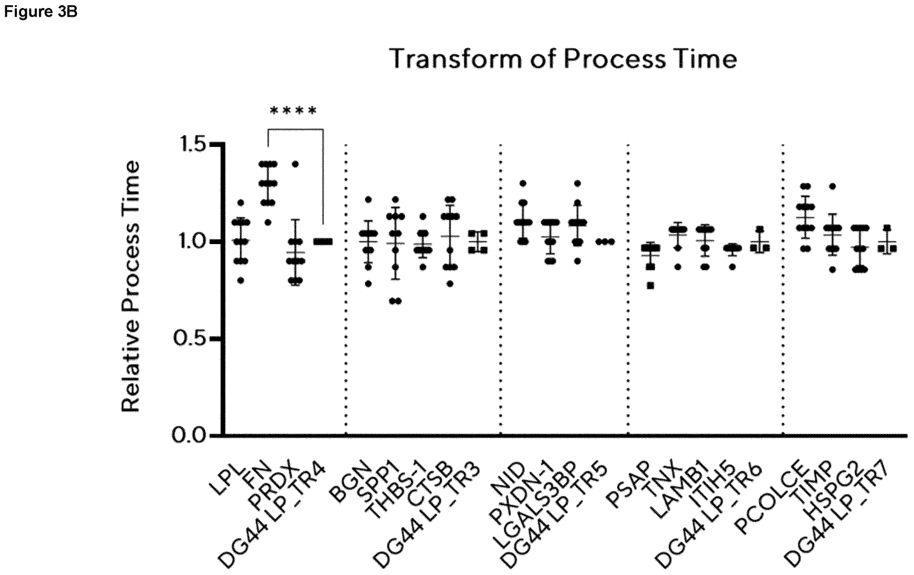

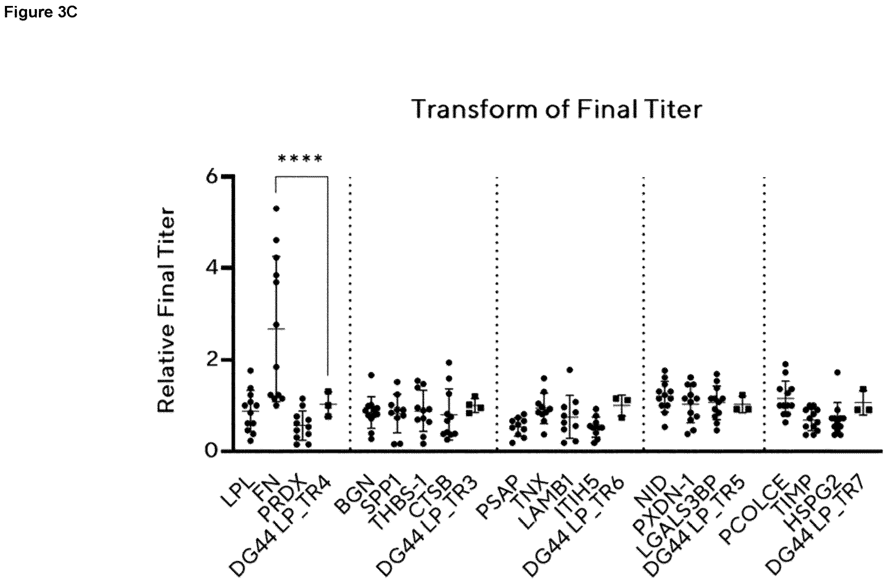

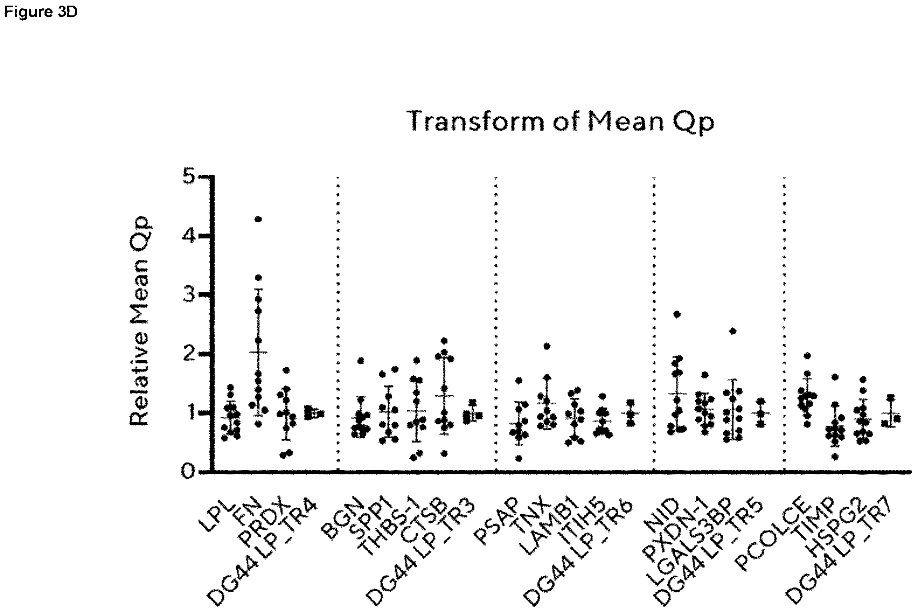

Figure 3A to D (Fig. 3A to D) shows: **Fig. 3A** the transform of Peak VCC (= viable cell concentration) by plotting the relative peak VCC (y-axis) determined for the generated clones detailed in the Examples (cf. **Example 3**) for the HCP targets as well as a respective CHO DG44 wild-type (WT) controls (TR4, TR3, TR5, TR6 and TR7, respectively - see also **Example 3** for further explanations). Fed-Batch endpoint is shown. **Fig. 3B**: Transform of process time. **Fig. 3C**: Transform of Final Titer on harvest day. **Fig. 3D**: Transform of mean Qp. A process ends once the viability falls below a pre-set threshold. Here, final viability, final titer [g/L] and final Qp [pg/c/d] are relevant. The cell specific productivity Qp is calculated using the cumulative integral of viable cell concentration (IVCC [×10⁵ cells*d/ml]) and titer for each day, respectively. Therefore, IVCC, process time and Qp describe the course of the bioprocess and its productivity. From the Qp, mean Qp is calculated, averaged over the time of cultivation. (Brown-Forysthe and Welch ANOVA test performed, Dunnett's test, alpha = 0.05)
**Figure 4** (Fig. 4A to D) shows the results of the experiments detailed in **Example 4** below, always measured for the 10 target HCPs knocked-out as detailed in this Example and the wild-type (WT) control. **Fig. 4A**: shows the peak VCC (viable cell concentration in [×10⁵ cells*/ml]) and the KO score (in%). **Fig. 4B**: shows the process time (in days) and the KO score (in%). **Fig. 4C**: shows the final titer (in g/L) and the KO score (in%). **Fig. 4D**: shows final Qp [pg/c/d] and the KO score (in%). The KO score is shown in bars, the further parameter according to Fig. 4A to D is reflected by the dots and the corresponding standard deviation.

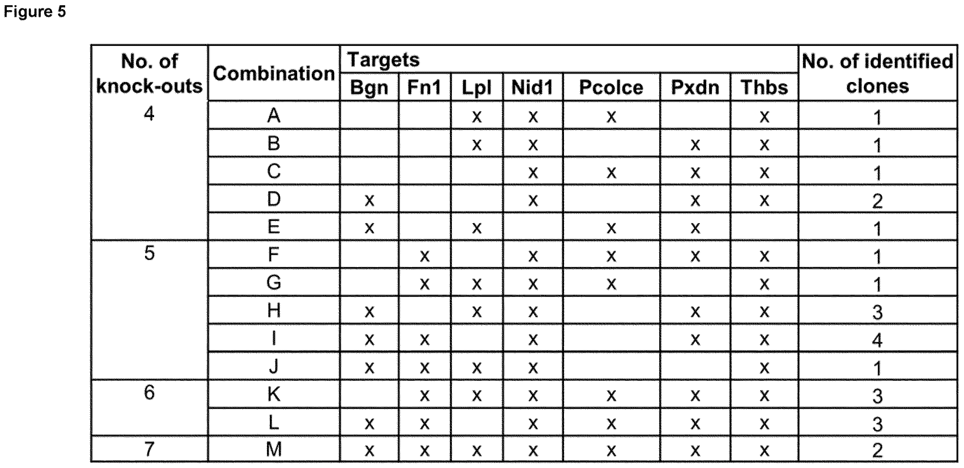

**Figure 5** (cf. **Example 5**) shows a matrix of multiple knock-out combinations (number / no. = 4, 5, 6, or 7) of the seven targets tested in **Example 5** and the number of identified clones.

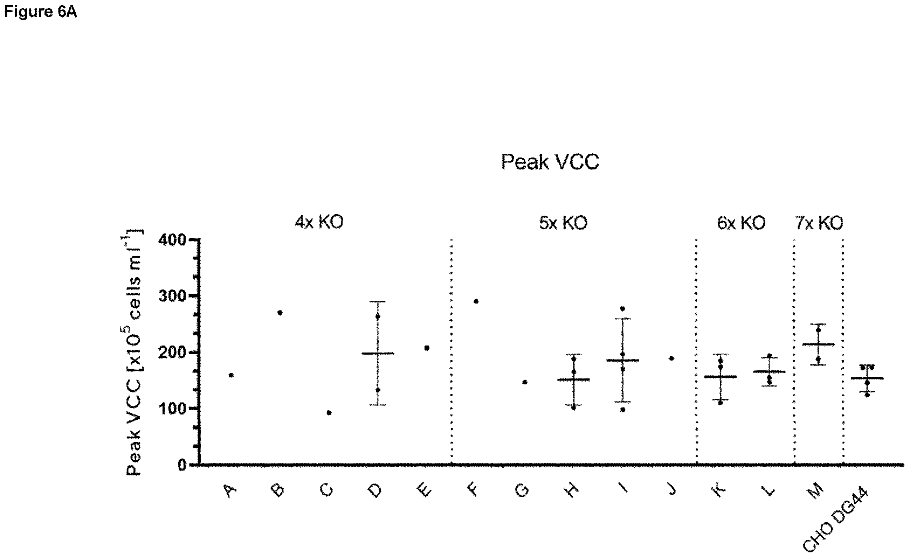

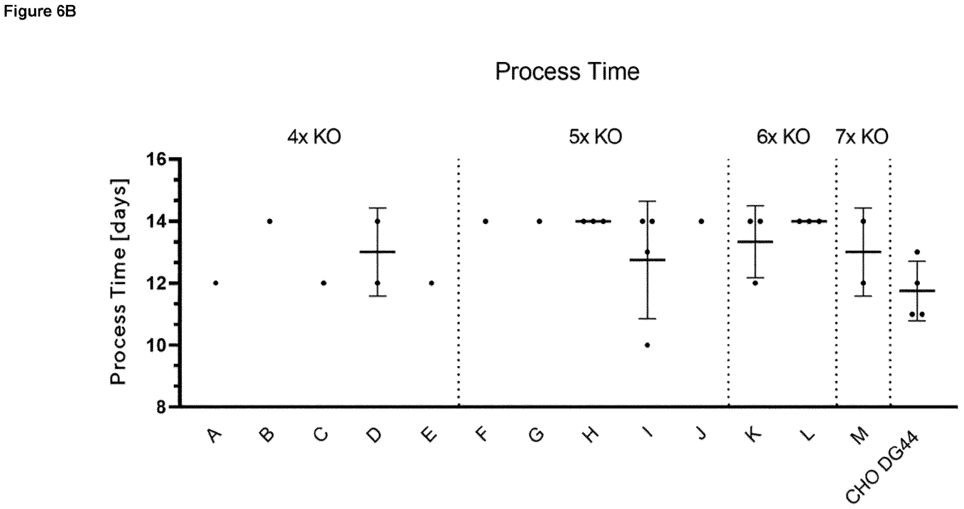

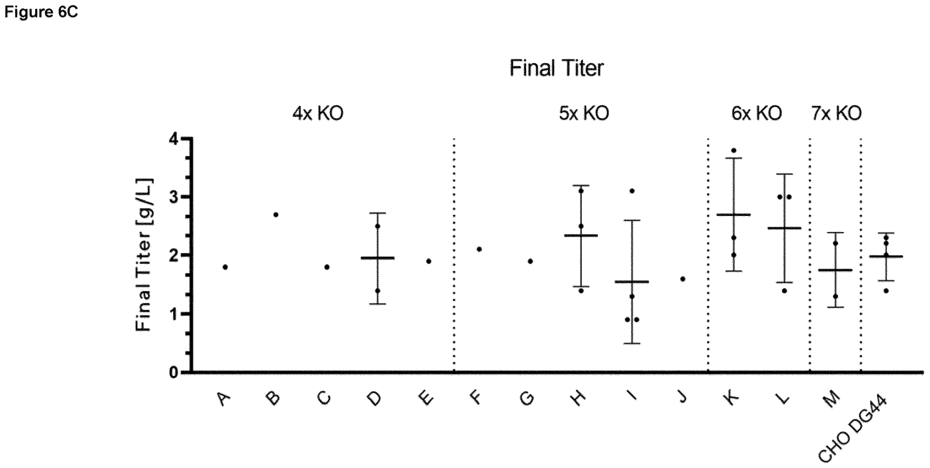

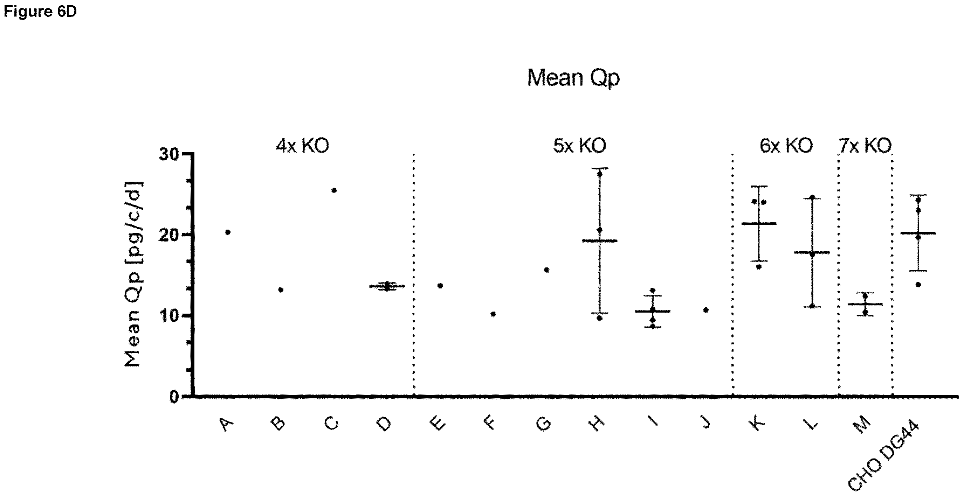

**Figure 6** (Fig. 6Ato D) shows the results of the experiments detailed in **Example 5.** All data are presented for the 4-fold, 5-fold, 6-fold or 7-fold KO as detailed in the overview matrix in **Figure 5****.** CHO DG44 served as control for all assays. **Fig. 6A**: shows the peak VCC (viable cell concentration in [×10⁵ cells*/ml]). **Fig. 6B**: shows the process time (in days). **Fig. 6C**: shows the final titer (in g/L). Fig. 46: shows the mean Qp [pg/c/d].

### Brief Description of Sequences

| **SEQ ID NO:** | **Description** |
|---|---|
| 1 | G3HHV4_Thrombospondin-1 (=THBS-1) |
| 2 | G3I1V3_Fibronectin (= FN) |
| 3 | G3HPV7_Histone H4 |
| 4 | G3H3Q1_Pyruvate kinase (=PKM) |
| 5 | G3HNJ3_Clusterin |
| 6 | G3GYP9_Peroxiredoxin-1 (= PRDX-1) |
| 7 | P17244_Glyceraldehyde-3-phosphate dehydrogenase |
| 8 | G3GVD0_Actin beta |
| 9 | G3I1Y9_Sulfated glycoprotein 1 (=PSAP) |
| 10 | G3H0E4_Chondroitin sulfate proteoglycan 4 (=CSPG4) |
| 11 | G3I255_L-lactate dehydrogenase (=LDHA) |
| 12 | G3HIM1_Basement membrane-specific heparin sulfate proteoglycan core protein (=HSPG-2) |
| 13 | G3IAQ0_phosphopyruvate hydratase |
| 14 | A0A3L7IKX6_Lipoprotein lipase (= LPL) |
| 15 | G3HHR3_Vimentin (=VIM) |
| 16 | G3I8R9_Endop!asmic reticulum chaperone (=HSPA5) |
| 17 | P19378_Heat shock cognate 71 kDa protein |
| 18 | G3H533_Peptidyl-prolyl cis-trans isomerase (=PPIB) |
| 19 | P09445_Elongation factor 2 |
| 20 | G3H0L9_Cathepsin B (=CTSB) |
| 21 | P46633_Heat shock protein HSP 90-alpha (=HSP90AA1) |
| 22 | G3I3Y6_Glutathione S-transferase |
| 23 | P62629_Elongation factor 1-alpha 1 |
| 24 | G3I3U5_Nidogen-1 (=NID or NID-1 or NID1) |
| 25 | G3GUU5_Transketolase |
| 26 | G3I129_Ubiquitin |
| 27 | G3IBF4_Serine_protease |
| 28 | G3HWE4_Nidogen-1 (=NID or NID-1 or NID1) |
| 29 | G3HBI1_Peroxidasin (=PXDN-1) |
| 30 | G3HKZ1_14-3-3 protein zeta/delta |
| 31 | G3H8V5_Carboxypeptidase |
| 32 | G3HIQ1_Peptidyl-prolyl cis-trans isomerase |
| 33 | G3IKC3_glutathione_transferase |
| 34 | G3HQM6_Endoplasmin |
| 35 | P50310_Phosphoglycerate kinase 1 |
| 36 | A0A061 HUR7_Biglycan (=BGN) |
| 37 | G3I1H5_Legumain |
| 38 | G3H3E4_Galectin-3-binding protein (=LGALS3BP) |
| 39 | G3I6T1_Phospholipase B-like |
| 40 | G3INX0_Histone H2B |
| 41 | G3H1W4_Tubulointerstitial nephritis antigen like 1 |
| 42 | G3!278_Laminin subunit beta-1 (=LAMB-1; LAMB1) |
| 43 | G3HBD4_Nucleoside diphosphate kinase |
| 44 | G3GR64_Inter-alpha-trypsin inhibitor heavy chain H5 (=ITIH5) |
| 45 | G3H7I6_Sulfhydryl oxidase |
| 46 | G3IF52_Nucleobindin-2 |
| 47 | Q9EPP7_Cathepsin X |
| 48 | G3HZD1_Tenascin-X (=TNX) |
| 49 | G3IG05_Annexin |
| 50 | G3IBK2_Filamin-A |
| 51 | G3HDT6_Histone H2A type 1 |
| 52 | G3I4H6_Fructose-bisphosphate aldolase |
| 53 | G3HH30_Aldo-keto reductase family 1 member B |
| 54 | G3HCX8_Calreticulin (= Calr) |
| 55 | G3I4Z7_Galectin (= Lgals1) |
| 56 | G3I4E8_Fatty_acid-binding protein |
| 57 | P68361_Tubulin alpha-1B chain |
| 58 | G3IF80_Nucleolin |
| 59 | G3H8F4_Dystroglycan 1 |
| 60 | G3HLV6_ATP_citrate synthase |
| 61 | G3HMV7_Alpha-L-fucosidase |
| 62 | G3HGW6_Laminin subunit alpha-5 (=LAMA5) |
| 63 | G3HC84_Heat shock protein HSP 90-beta |
| 64 | G3GXD7_Fatty acid synthase |
| 65 | G3HQP8_Tubulin beta chain |
| 66 | G3IKQ9_Osteopontin (=SPP1) |
| 67 | G3IIE7_Procollagen-lysine, 2-oxoglutarate 5-dioxygenase |
| 68 | G3IBH0_Metalloproteinase inhibitor 1; Tissue inhibitor of metalloproteinases 1(=TIMP1) |
| 69 | G3IHY5_6-phosphogluconate dehydrogenase |
| 70 | G3ILK7_Calsyntenin-1 |
| 71 | G3HC31_Protein S100 |
| 72 | G3HKG9_60S acidic ribosomal protein |
| 73 | G3HDQ2_Malate dehydrogenase |
| 74 | G3HLK3_Granulins |
| 75 | G3I664_Procollagen C-endopeptidase enhancer 1 (=PCOLCE) |
| 76 | G3HMA1_Adipocyte enhancer-binding protein 1 (=AEBP1) |
| 77 | G3H4T5_Glypican-1 (=Gpc1) |
| 78 | G3HAI3_Follistatin-related protein 1 (=FSTL1) |
| 79 | G3H0U6_protein disulfide-isomerase |
| 80 | G3IHE5_GTP-binding nuclear protein |
| 81 | G3IEY9_14-3-3 protein theta |
| 82 | G3I973_Hypoxia up-regulated 1 |
| 83 | G3IE48_Elongation factor 1-gamma |
| 84 | G3HDS3_Histone H2A |
| 85 | G3HPV6_Histone H4 |
| 86 | G3HQX0 40S ribosomal protein |
| 87 | G3I6I6_Tubulin alpha chain |
| 88 | G3IED5_Peptidyl-prolyl cis-trans isomerase A |
| 89 | G3IEG6_Tubulin beta chain |
| 90 | A0A3L7HQ14_Eukaryotic translation initiation factor 3 subunit B |
| 91 | G3HC64_FAD synthase |
| 92 | A0A3L7I8J7_Leucine zipper protein 1 |
| 93 | G3IDD4_Serpin H1 |
| 94 | A0A3L7I8K8_Protein Wnt (=Wnt4 or WNT-4) |
| 95 | G3I8B5_Matrix metalloproteinase-28 (= Mmp28) |
| 96 | A0A8C2LJ19_Sparc/osteonectin (= Sparc) |
| 97 | A0A8C2N4D5_protein disulfide-isomerase |
| 98 | A0A8C2MC57_Transforming growth factor beta (= Tgfb1) |
| 99 | A0A8C2N0F8_Trinucleotide repeat containing 6C |
| 100 | G3GXY1_Beta-catenin-like protein 1 |
| 101 | G3GXZ0_Protein-glutamine gamma-glutamyltransferase 2 (= Tgm2) |
| 102 | A0A8C2LRW3_Catenin delta 1 |
| 103 | G3HFS5_Transforming growth factor beta-3 proprotein (=Tgfb3) |
| 104 | A0A8C2LYC0_Laminin subunit gamma 1 (= Lamc1) |
| 105 | A0A8C2QIZ6_Dynein cytoplasmic 1 heavy chain 1 |
| 106 | A0A8C2QLM9_Collagen type VII alpha 1 chain (=Col7a1) |
| 107 | G3H996_Catenin beta-1 |
| 108 | A0A8C2MMI8_A disintegrin-like and metallopeptidase (=Adamts-1) |
| 109 | A0A8C2M328_ADAM metallopeptidase (=Adamts-7) |
| 110 | A0A8C2M6G9_Trinucleotide repeat containing 6a |
| 111 | O55058_EGF-containing fibulin-like extracellular matrix protein 2 (= Efemp2) |
| 112 | A0A8C2LVZ9_Milk fat globule-EGF factor 8 protein (= Mfge8) |
| 113 | A0A8C2MY83_Latent transforming growth factor beta binding protein 3 (= Ltbp3) |
| 114 | G3ICW1_Osteoclast-stimulating factor 1 |
| 115 | G3HAF4_Splicing factor 3B subunit 3 |
| 116 | A0A8C2M0V0_Transforming growth factor beta 1 induced transcript 1 |
| 117 | G3IHE0_Cyclic AMP-dependent transcription factor |
| 118 | G3IK13_Eukaryotic translation initiation factor 3 subunit C |
| 119 | Q8K3H7_Calreticulin |
| 120 | P38982_40S ribosomal protein SA |
| 121 | G3HW00_Trinucleotide repeat-containing gene 6B protein |
| 122 | A0A8C2M0N5 Catenin alpha like 1 |
| 123 | G3HEY9_Catenin_alpha-1 |
| 124 | A0A8C2M1I7_Collagen alpha-2(V) chain (=Col5a2) |
| 125 | A0A8C2MW41_Diaphanous related formin 1 |
| 126 | G3H4H3_Calreticulin |
| 127 | G3H6V7_ Lipoprotein lipase (= LPL) |
| 128 | G3HSX8_ Biglycan |
| 129 | LPL_HDR_gRNA1 all guide RNAs: crRNA provided 5'→3' (without PAM sequence) |
| 130 | LPL_HDR_gRNA2 |
| 131 | LPL_HDR_gRNA3 |
| 132 | CTSB-E2-gRNA1 |
| 133 | CTSB-E2-gRNA2 |
| 134 | CTSB-E2-gRNA3 |
| 135 | LPL2_gRNA1 |
| 136 | LPL2_gRNA2 |
| 137 | LPL2_gRNA3 |
| 138 | Bgn_gRNA1 |
| 139 | Bgn_gRNA2 |
| 140 | Bgn_gRNA3 |
| 141 | Fn1_gRNA1 |
| 142 | Fn1_gRNA2 |
| 143 | Fn1_gRNA3 |
| 144 | PRDX1_gRNA1 |
| 145 | PRDX1_gRNA2 |
| 146 | PRDX1_gRNA3 |
| 147 | Psap_gRNA1 |
| 148 | Psap_gRNA2 |
| 149 | Psap_gRNA3 |
| 150 | Spp1_gRNA1 |
| 151 | Spp1_gRNA2 |
| 152 | Spp1_gRNA3 |
| 153 | Thbs_gRNA1 |
| 154 | Thbs_gRNA2 |
| 155 | Thbs_gRNA3 |
| 156 | A0A3L7HVW3 Nidogen-1 (Nid-1, NID) |

### Definitions

An "ECM" protein as used herein refers to a protein or part thereof (extracellular; transmembrane and/or intracellular) forming an extracellular matrix (ECM) by which a cell, particularly a CHO cell, is surrounded. Proteins as well as proteins involved in the overall genesis of ECM proteins, including the regulation, processing assembly and membrane insertion of ECM proteins or their intracellular/membrane-bound counterparts stabilizing and/or allowing the full signaling and/or function of the directly membrane-associated ECM are referred to as "ECM-related (or associated) proteins" herein. Notably, as explained in **Example 1** in comparison to **Example 2** below, the determination of ECM and ECM-related proteins followed a completely different experimental set-up: therefore, different production clones and conditions were used, and completely different quantification and analysis strategies were used. Basically, the ECM and ECM-related protein exploration process followed a transcriptome-based approach, whereas the acHCP and drHCP definition followed a peptide analysis and comparison-based approach. This is understandable before the background of the data results of interest: for the acHCP/drHCP project, the total quantifiable amount of HCPs was of major interest, whereas the ECM/ECM-related project aimed at defining a subset of metabolic burden proteins, so that the quantification was already done on the level of transcription, said process already representing an energy intensive step during recombinant protein production that the present inventors explored to optimize in a straight manner based on the findings obtained.

An "antibody fragment" or any "(functional) fragment" of an "antibody" as used herein refers to a molecule other than a full-length antibody that comprises at least one portion of an intact antibody that binds the antigen to which the full-length antibody binds. Examples of antibody fragments may include, but are not limited to, Fv, Fab, Fab', Fab'-SH, F(ab')2; diabodies; linear antibodies; single-chain antibody molecules (e.g., scFv, and scFab); single domain antibodies (dAbs); and/or multispecific antibodies which assemble from antibody fragments.

In the context of an "antibody" or a "fragment" thereof, the term "chimeric" refers to an antibody in which a portion of the heavy and/or light chain originates from a particular source or species, while the remainder of the heavy and/or light chain is derived from a different source or species. As it is known to one skilled in the art, the "class" of an antibody refers to the type of constant domain or constant region of its cognate heavy chain. There are five major classes of antibodies: IgA, IgD, IgE, IgG and IgM, and several of these can be further divided into subclasses (isotypes), e.g., IgG1, IgG2, IgG3, IgG4, IgAl, and IgA2. In certain embodiments, the antibody is of the IgG 1.

The terms "abundant core HCP" (acHCP), "abundant HCP" and "core HCP" are used interchangeably herein. The terms denote those HCPs that (cf. **Fig. 2** and **Table 2**) occur abundant in a host cell of interest in the sense that the HCP is present at the day of harvest and/or is present throughout the cultivation process until harvest, and particularly at the time point of harvest, in a substantial amount, usually a quantifiable amount. Notably, the definition of a core HCP inherently depends on the fact that is it is present above the Lower Limit of Quantification (LLOQ). Still, the skilled person is aware of the fact that the LLOQ may vary depending on the quantification means and the assay conditions used. Further, the LLOQ may be even lower with more sensitive devices and techniques being developed. Therefore, an acHCP as used herein may also refer to an acHCP below the present quantification limit (cf. **Fig. 2B**), if it fulfills the further criterion of being present substantially throughout the complete cultivation process and particularly at the time of harvest. See also **Example 2.**

The term "difficult-to-remove HCP" or "drHCP" as used herein refers to a HCP that is present in a quantifiable amount in at least one sample after harvest and after at least one further purification step. Particularly, the HCP is still present in at least one of the sample shown in the right column in **Fig. 1**, i.e., the HCP cannot be removed by at least one of the purification strategies used after harvest. See also **Example 2** for a further explanation and the list provided in **Table 3.**

The term "downstream processing" or "DSP" as used herein, e.g. in the context of cell cultures, refers to the entirety of process steps required for obtaining a recombinantly produced molecule in purified form from a host cell suspension, wherein the cell harvest as the end of the respective bioprocess marks the starting point of the downstream processing as opposed to the upstream process.

"Harvesting" in this context implies the endpoint of the culture of the host cell for obtaining a recombinant protein, including a recombinant peptide, of interest.

The term "target nucleic acid segment" as used herein refers to a nucleotide sequence, typically a DNA sequence, which can be subjected to modification using a site-directed endonuclease. Typically, a target nucleic acid segment is part of a genome.

Accordingly, the term "upstream processing" or "USP" as used herein, e.g. in the context of cell cultures refers to the entirety of cultivation steps taking place prior to the cell harvest.

The term "upstream processing performance" as used herein refers to an evaluation of the upstream processing of a given cell culture based on one or more parameters, wherein said parameters may be selected from peak viable cell concentration (VCC), integral viable cell concentration (IVCC), cell specific productivity, the final titer, and total bioprocess time. Generally, high values determined for any of the aforementioned parameters indicate a good upstream processing performance. Other parameters may also be assessed for evaluating the upstream processing performance of a given cell culture.

The term "expression" as used herein refers to the process of transcription (e.g., for a functional gRNA) and translation (to achieve a polypeptide) within a host cell. The level or degree of expression of a product gene to the cognate RNA in a host cell can be determined on the basis of either the amount of corresponding mRNA that is present in the cell or the amount of the protein encoded by the product gene that is produced by the cell. For example, mRNA transcribed from a product gene is desirably quantitated by northern hybridization (see, e.g., Sambrook et al., Molecular Cloning: A Laboratory Manual, pp. 7.3-7.57 (Cold Spring Harbor Laboratory Press, 1989). The polypeptide or protein encoded by a product gene can be quantitated either by assaying for the biological activity of the protein or by using assays that are independent of such activity, such as western blotting using antibodies that are capable of reacting with the protein (see, e.g., Sambrook et al., Molecular Cloning: A Laboratory Manual, pp. 18.1-18.88 (Cold Spring Harbor Laboratory Press, 1989). A "reduced functional expression" as used herein in turn refers to the reduction and/or elimination of the expression of one or more endogenous products relative to the expression level of the endogenous product(s) in an unmodified cell. A reduction of expression may comprise a reduction and/or complete elimination of the functional endogenous product (on RNA and/or protein level).

The term "host cell proteins" (HCPs) as used herein refers to endogenous proteins produced by host cell lines, possibly engineered host cell lines, at varying levels. In case Chinese Hamster Ovary (CHO) cells are used as host cell line, host cell proteins (HCPs) are those proteins, which are produced by CHO cells and which are endogenous to CHO cells.

The term "peptibody" as used herein refers to a part or all of an antibody fused to a peptide. Thus, peptibodies are usually characterized by a combination of the activity of the respective peptide with the longer duration of activity of an antibody.

The terms "polypeptide" and "protein" are used interchangeably herein and refer to a contiguous stretch of amino acid sequences defining the primary structure of the polypeptide. A properly folded protein can have structural (e.g., matrix protein), or functional activity, e.g. enzymatic activity / functional activity, or it can have specific binding or recognition properties (e.g., an antibody), or a combination thereof (binding + nuclease activity, e.g. CRISPR nuclease).

The "recombinant protein of interest" as used herein may be any protein, peptide or fragment thereof that can be produced when exogenously added to a host cell, usually as recombinant (i.e., foreign) DNA or RNA, e.g., on a plasmid, as a viral vector, or as transgene, for example, as introduced via genome editing and the like. Further, a recombinant protein may also be a protein encoded by an endogenous gene that was modified via a genome editing technology, including base editing, prime editing, and InDel induced by genome editing and the like. A recombinant protein of interest can be a therapeutic protein, peptide or fragment thereof, or any other protein, peptide or fragment thereof. The recombinant protein may be an antibody or a variant or functional fragment thereof as defined above, or any other prophylactic or therapeutic protein. Any other protein, e.g., a fluorescent protein like EGFP, is also to be understood as recombinant protein, as long as the protein or fragment thereof has been inserted into, has been mutated, and/or has been artificially created in a host cell of interest.

The terms "RNA interference" or "RNAi" or "RNA silencing" or "gene silencing" as used herein interchangeably refer to a gene down-regulation (or knock-down) mechanism meanwhile demonstrated to exist in all eukaryotes. The mechanism was first recognized in plants where it was called "post-transcriptional gene silencing" or "PTGS". In RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and an antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". In an RNA hairpin, sense sequences and corresponding antisense sequences, together form a double strand and are separated by an "intervening intron loop sequence" forming the loop of the hairpin structure. An "RNAi agent" as used herein may also comprise more than one sense and antisense pair and form several loops.

In the tables shown herein, for some proteins more than one Uniprot ID is given, wherein in each case the first Uniprot ID is the preferred one, which is to be considered in case a distinction of one or more proteins listed in any of the respective Tables from any other protein based on their respective Uniprot ID is required. All Uniprot IDs listed for any of the proteins in the respective Tables correspond to the Uniprot IDs of the respective proteins on the date of filing of this application. The respective Tables are to be understood such that they also include all homologues, orthologues, or paralogues of all proteins explicitly contained in any of the respective Tables. For certain homologues, orthologues, or paralogues individual SEQ ID NOs are provided (e.g., SEQ ID Nos: 14 and 127), for others, the UniProt ID is provided along with one exemplary (but not restricting) SEQ ID NO showing the respective reference sequence.

A "therapeutic molecule" or "biotherapeutic molecule or agent" as used herein refers to a recombinant protein of interest as defined above, or any other recombinant nucleic acid molecule that has a prophylactic or curative effect when used to treat a subject in need thereof.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/emboss_water/) nucleic acids or the EMBOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

The present invention overcomes the needs in the art by providing methods for creating generally applicable ECM protein and ECM-related protein expression profiles and based on this finding optimized CHO cell lines in which single or multiple ECM or ECM-related proteins are knocked-out resulting in a reduction of the metabolic burden and/or an improved bioprocess performance. This results, *inter alia*, in a significant improvement of one or several of the pivotal bioprocess parameters selected from the group consisting of viable cell concentration, specific productivity, final titer and cell culture longevity/process duration. Additionally, the methods can be favorably combined with multiple knock-outs to achieve an overall reduction of the content of HCPs in a final product.

For the first time, the present inventors used extensive transcriptome profiling assays to identify a panel of transcripts encoding ECM and ECM-related proteins to identify transcriptome patterns corresponding to HCP expression profiles in a host cell in a first step to establish a sound data base for determining which of the ECM or ECM-related proteins are expendable or even obstructive for the production of recombinant biotherapeutic drugs in CHO cells, as this class of proteins is not necessarily needed for a CHO cell in suspension culture, but rather represent a metabolic burden representing remnants of the cell's origin as adhesive cell. Systematic and labor-intensive comparisons of transcriptome and expression profile studies have thus been performed for the purpose of the present invention to provide a generally applicable platform host cell, preferably a modified CHO cell.

The major findings confirmed that a CHO cell with a significantly reduced ECM or ECM-related protein profile shows a maintained or even improved overall product yield and upstream and downstream processing parameters during recombinant biotherapeutic production. Simultaneously, this modified cell shows a reduced HCP profile, as any expendable ECM at the same time represents an HCP undesirable as co-purifying contaminant.

As a basis for the identification of relevant ECM or ECM-related knock-out targets, several mAb production clones were processed under industrial USP (ambr250 fed-batch process) conditions as listed in **Fig. 1A**. Cell samples were taken throughout the whole process on a daily basis, RNA was extracted, subjected to RNA-seq analysis and transcriptome profiles were generated. This high-throughput screenings and the comparison and evaluation of multiple data sets thus ultimately allowed the identification of ECM and ECM-related proteins as highly interesting targets for knock-outs to optimize CHO cells, as the ECMs themselves as well as the related or associated proteins contributing to ECM genesis and/or function are not necessarily needed for nowadays CHO cells already fine-tuned for recombinant molecule production. ECM and ECM-related proteins, as such evolutionary conserved and still substantially expressed in commercial CHO cell lines were thus surprisingly found to be expendable to a large extent for exactly that purpose CHO cells are primarily cultured for: high yield recombinant protein production with a streamlined bioprocess performance. Therefore, the findings as presented herein can help to significantly improve bioprocess performance and to increase production yields, whilst simultaneously reducing costs for purification steps.

In a first aspect, the present invention provides a CHO cell with a modified extracellular matrix (ECM) profile, characterized by a reduced load of at least one endogenous protein being an ECM protein or an ECM-related protein, wherein said CHO cell comprises: at least one modification in at least one endogenous coding sequence coding for at least one endogenous ECM protein or endogenous ECM-related protein selected from the group summarized in **Table 1**, or of any homologue, orthologue, or paralogue thereof, and optionally at least one further modification, preferably, wherein the modification leads to a knock-down or a knock-out of expression, meaning a significantly reduced or even abolished transcription and/or translation, respectively.

**Table 1**

| **No.** | **Protein name** | **Uniprot ID** | **SEQ ID NO:** |
|---|---|---|---|
| 1 | Fibronectin | G3I1V3 A0A3L7IDB0 | 2 |
| 2 | Basement membrane-specific heparan sulfate proteoglycan core protein | G3HIM1 A0A3L7I8L8 | 12 |
| 3 | Peroxidasin | G3HBI1 A0A3L7HCC3 | 29 |
| 4 | Laminin subunit beta-1 | G3I278 A0A3L7HMC9 | 42 |
| 5 | Calreticulin | G3HCX8 | 54 |
| 6 | Galectin | G3I4Z7 A0A3L7I2M5 | 55 |
| 7 | Glypican-1 | G3H4T5 A0A3L7IDU1 | 77 |
| 8 | Cyclic AMP-dependent transcription factor ATF-4 | G3IHE0 | 117 |
| 9 | A disintegrin and metalloproteinase with thrombospondin motifs 1 | A0A8C2MMI8 | 108 |
| 10 | A disintegrin and metalloproteinase with thrombospondin motifs 7 | A0A8C2M328 | 109 |
| 11 | Calreticulin | Q8K3H7 | 119 |
| 12 | Calreticulin-3 | G3H4H3 | 126 |
| 13 | Collagen alpha-2(V) chain | A0A8C2M1I7 | 124 |
| 14 | Collagen alpha-1(VII) chain | A0A8C2QLM9 | 106 |
| 15 | Catenin alpha-1 | G3HEY9 | 123 |
| 16 | Alpha-catulin | A0A8C2M0N5 | 122 |
| 17 | Catenin beta-1 | G3H996 | 107 |
| 18 | Beta-catenin-like protein 1 | G3GXY1 | 100 |
| 19 | Catenin delta-1 | A0A8C2LRW3 | 102 |
| 20 | Protein diaphanous homolog 1 | A0A8C2MW41 | 125 |
| 21 | Cytoplasmic dynein 1 heavy chain 1 | A0A8C2QIZ6 | 105 |
| 22 | EFEMP2 | O55058 | 111 |
| 23 | Eukaryotic translation initiation factor 3 subunit B | A0A3L7HQ14 | 90 |
| 24 | Eukaryotic translation initiation factor 3 subunit C | G3IK13 | 118 |
| 25 | FAD synthase | G3HC64 | 91 |
| 26 | Laminin subunit gamma-1 | A0A8C2LYC0 | 104 |
| 27 | Latent-transforming growth factor beta-binding protein 3 | A0A8C2MY83 | 113 |
| 28 | Leucine zipper protein 1 | A0A3L7I8J7 | 92 |
| 29 | Lactadherin | A0A8C2LVZ9 | 112 |
| 30 | Matrix metalloproteinase-28 | G3I8B5 | 95 |
| 31 | Osteoclast-stimulating factor 1 | G3ICW1 | 114 |
| 32 | RPSA | P38982 | 120 |
| 33 | Serpin H1 | G3IDD4 | 93 |
| 34 | Splicing factor 3B subunit 3 | G3HAF4 | 115 |
| 35 | Transforming growth factor beta-1 proprotein | A0A8C2MC57 | 98 |
| 36 | Transforming growth factor beta-1-induced transcript 1 protein | A0A8C2M0V0 | 116 |
| 37 | Transforming growth factor beta-3 proprotein | G3HFS5 | 103 |
| 38 | Protein-glutamine gamma-glutamyltransferase 2 | G3GXZ0 | 101 |
| 39 | Trinucleotide repeat-containing gene 6A protein | A0A8C2M6G9 | 110 |
| 40 | Trinucleotide repeat-containing gene 6B protein | G3HW00 | 121 |
| 41 | Trinucleotide repeat-containing gene 6C protein | A0A8C2N0F8 | 99 |
| 42 | Thioredoxin domain-containing protein 5 | A0A8C2N4D5 | 97 |
| 43 | Protein Wnt-4 | A0A3L7I8K8 | 94 |

Surprisingly, the transcriptome, bioinformatics and big data-based analysis exploration approach as used to identify the major ECM and ECM-related proteins causing a high metabolic burden to a host cell (cf. **Example 1, 1.1** to **1.4**) yielded a highly interesting panel or profile of proteins that represent hot spots for mutational studies to reduce the transcription and translation efforts of a cell, especially in view of the fact that the specific approach used did not exclusively yield candidates like collagen etc. known as ECMs as the most prominent candidates. The approach rather elucidated a network of proteins all involved in ECM-genesis and thus represents a highly promising basis for efficient ECM and ECM-related protein targeting modification studies. An ECM profile as used herein thus refers to at least one, preferably more than one, ECM or ECM-related protein that was identified according to the transcriptome profiling methods disclosed herein that turned out to be (a) highly valuable candidate(s) for a targeted knock-out to reduce the overall metabolic burden of a CHO cell to actively shape the genome of said CHO cell towards a better performance in the recombinant production of biopharmaceuticals.

Functional single and multiple knock-out studies performed surprisingly demonstrated that ECM and ECM-related proteins as shown in **Table 1** above, alone or in combination with other HCPs, are excellent candidates for the creation of engineered CHO cell lines with a reduced metabolic burden according to the invention as single knock-outs performed did not show significant negative or positive impacts on the performance parameters, including, for example, performance parameters like peak viable cell concentration, specific productivity, final titer and process duration etc., whereas certain candidates, including a knock-out of Fibronectin (Fn1) qualifying as ECM, acHCP and drHCP protein at the same time according to the present invention surprisingly even had a significant positive impact on final titer and process duration/cell culture longevity (cf. **Example 1, 1.1** to **1.4** (focusing on ECM and ECM-related proteins), and **Example 3** and **5** (both **Examples 3** and **5** focusing on ECM, ECM-related and acHCP and drHCP targets) and **Figure 3** **A to D**).

Therefore, the present inventors succeeded in providing a guidance in selecting individual and combined knock-out targets and panels for creating engineered CHO cell lines with a reduced metabolic burden, optionally also showing a significantly reduced HCP content favorable for recombinant protein production.

In preferred embodiments, the at least one further modification may be present in at least one endogenous coding sequence, which codes for an HCP being highly abundant (acHCPs). The acHCP may simultaneously represent an ECM or an ECM-related protein as defined and used herein. **Table 2** summarizes a list of highly abundant acHCPs, for which the mean relative concentration, named "MRC" in **Table 2** below, was calculated for each individual HCP by determining the mean value of all measured concentrations (in ng/mL) over the sum of all probes measured. All mean values were added to each other and for each HCP, the mean value was divided by the mean HCP total concentration. The resulting value corresponds to the "mean relative concentration" or MRC as used herein.

Modifying, i.e., reducing, the amount of at least one acHCP expressed in a cell with a high mean relative concentration meaning a concentration above 0.05%, preferably above 0.1%, even more preferably 0.15% and above will lead to a significant decrease of the overall HCP load. This is particularly true for acHCPs with a very high mean relative concentration meaning a concentration above 0.5%, preferably above 1%, even more preferably with a mean relative concentration of 1.5% and above.

**Table 2**

| Protein name | Uniprot ID | Location | MRC |
|---|---|---|---|
| Thrombospondin-1 | G3HHV4 | extracellular | 6,54% |
| Fibronectin | G3I1V3 | extracellular | 4,53% |
| | A0A3L7IDB0 | | |
| Histone H4 | G3HPV7 | intracellular | 2,72% |
| Pyruvate kinase | G3H3Q1 | intracellular | 2,68% |
| Clusterin | G3HNJ3 | extracellular | 2,49% |
| Peroxiredoxin-1 | G3GYP9 | intracellular | 2,36% |
| Glyceraldehyde-3-phosphate dehydrogenase | P17244 | intracellular | 2,14% |
| | G3HMD1 | | |
| Actin beta | G3GVD0 | intracellular | 1,85% |
| Sulfated glycoprotein 1 | G3I1Y9 | extracellular | 1,83% |
| Chondroitin sulfate proteoglycan 4 | G3H0E4 | plasma membrane | 1,83% |
| L-lactate dehydrogenase | G3I255 | intracellular | 1,55% |
| Basement membrane-specific heparan sulfate proteoglycan core protein | G3HIM1 | plasma membrane | 1,54% |
| | A0A3L7I8L8 | | |
| Phosphopyruvate hydratase | G3IAQ0 | intracellular | 1,46% |
| Lipoprotein lipase | G3H6V7 | extracellular | 1,31% |
| | A0A3L7IKX6 | | |
| Vimentin | G3HHR3 | intracellular | 1,28% |
| Endoplasmic reticulum chaperone BiP | G3I8R9 | intracellular | 1,25% |
| Heat shock cognate 71 kDa protein | P19378 | intracellular | 1,24% |
| Peptidyl-prolyl cis-trans isomerase | G3H533 | intracellular | 1,22% |
| Elongation factor 2 | G3HSL4 | intracellular | 1,16% |
| | P09445 | | |
| Cathepsin B | G3H0L9 | intracellular | 1,15% |
| Heat shock protein HSP 90-alpha | P46633 | intracellular | 1,11% |
| Glutathione S-transferase | G3I3Y6 | intracellular | 1,00% |
| Elongation factor 1-alpha 1 | P62629 | intracellular | 0,96% |
| | G3HH39 | | |
| Nidogen-1 | G3I3U5 | extracellular | 0,84% |
| Transketolase | G3GUU5 | intracellular | 0,83% |
| Serine protease HTRA1 | G3IBF4 | extracellular | 0,78% |
| Nidogen-1 | G3HWE4 | extracellular | 0,76% |
| | A0A3L7HVW3 | | |
| Peroxidasin | G3HBI1 | extracellular | 0,69% |
| | A0A3L7HCC3 | | |
| 14-3-3 protein zeta/delta | G3HKZ1 | intracellular | 0,69% |
| Carboxypeptidase | G3H8V5 | extracellular | 0,66% |
| Glutathione transferase | G3IKC3 | intracellular | 0,66% |
| Endoplasmin | G3HQM6 | intracellular | 0,58% |
| Phosphoglycerate kinase 1 | P50310 | intracellular | 0,56% |
| Biglycan | G3HSX8 | extracellular | 0,55% |
| | A0A061HUR7 | | |
| Legumain | G3I1H5 | intracellular | 0,52% |
| Galectin-3-binding protein | G3H3E4 | extracellular | 0,50% |
| Phospholipase B-like | G3I6T1 | intracellular | 0,49% |
| Tubulointerstitial nephritis antigen-like | G3H1W4 | intracellular | 0,47% |
| Laminin subunit beta-1 | G3I278 | extracellular | 0,44% |
| | A0A3L7HMC9 | | |
| Nucleoside diphosphate kinase | G3HBD4 | intracellular | 0,43% |
| Inter-alpha-trypsin inhibitor heavy chain H5 | G3GR64 | extracellular | 0,41% |
| Sulfhydryl oxidase | G3H7I6 | extracellular | 0,40% |
| Nucleobindin-2 | G3IF52 | intracellular | 0,39% |
| Cathepsin X | Q9EPP7 | intracellular | 0,39% |
| Tenascin-X | G3HZD1 | extracellular | 0,38% |
| Annexin | G3IG05 | extracellular | 0,38% |
| Filamin-A | G3IBK2 | intracellular | 0,37% |
| Fructose-bisphosphate aldolase | G3I4H6 | intracellular | 0,37% |
| Aldo-keto reductase family 1 member B | G3HH30 | intracellular | 0,37% |
| Galectin | G3I4Z7 | extracellular | 0,36% |
| | A0A3L7I2M5 | | |
| Adipocyte-type fatty acid-binding protein | G3I4E8 | intracellular | 0,35% |
| Tubulin alpha-1B chain | P68361 | intracellular | 0,35% |
| Nucleolin | G3IF80 | intracellular | 0,35% |
| Dystroglycan 1 | G3H8F4 | plasma membrane | 0,33% |
| ATP citrate synthase | G3HLV6 | intracellular | 0,32% |
| Alpha-L-fucosidase | G3HMV7 | intracellular | 0,32% |
| Laminin subunit alpha-5 | G3HGW6 | extracellular | 0,30% |
| Heat shock protein HSP 90-beta | G3HC84 | intracellular | 0,29% |
| Fatty acid synthase | G3GXD7 | intracellular | 0,29% |
| Tubulin beta chain | G3HQP8 | intracellular | 0,28% |
| Procollagen-lysine,2-oxoglutarate 5-dioxygenase 1 | G3IIE7 | intracellular | 0,24% |
| Metalloproteinase inhibitor 1 | G3IBH0 | extracellular | 0,23% |
| Calsyntenin-1 | G3ILK7 | plasma membrane | 0,21% |
| Procollagen C-endopeptidase enhancer 1 | G3I664 | extracellular | 0,17% |
| Adipocyte enhancer-binding protein 1 | G3HMA1 | extracellular | 0,17% |
| Glypican-1 | G3H4T5 | plasma membrane | 0,16% |
| | A0A3L7IDU1 | | |
| Follistatin-related protein 1 | G3HAI3 | extracellular | 0,15% |

Further, in particular embodiments, it may be preferable that the at least one modification is present in at least one endogenous coding sequence, which codes for an HCP being difficult-to-remove (drHCP). **Table 3** below summarizes a list of drHCPs, for which the mean relative concentration, named "MRC" in **Table 3** below, was determined as detailed above for **Table 2**. As already defined above, a drHCP is one that is present at least after a protein A column purification (ProA), after virus inactivation (VI), after cation exchange chromatography (CEX), or even after anion exchange chromatography (AEX), when PrA, VI, CEX and AEX are conducted as a series of subsequent purification steps.

Therefore, modifying at least a drHCP, preferably at the same time being an acHCP, or modifying a mix of at least two drHCPs and acHCPs, or both, can significantly reduce the amount of HCPs in a targeted way by specifically defining a set of acHCPs and/or drHCPs in a first step to select and modify the right panel of candidates.

**Table 3**

| Protein name | Uniprot ID | Location | MRC | Pro A | VI | CEX | AEX |
|---|---|---|---|---|---|---|---|
| Thrombospondin-1 | G3HHV4 | extracellular | 6,54% | x | x | | |
| Fibronectin | G3I1V3 | extracellular | 4,53% | x | x | | |
| | A0A3L7IDB0 | | | | | | |
| Pyruvate kinase | G3H3Q1 | intracellular | 2,68% | x | x | | |
| Clusterin | G3HNJ3 | extracellular | 2,49% | x | x | x | |
| Peroxiredoxin-1 | G3GYP9 | intracellular | 2,36% | x | x | x | x |
| Glyceraldehyde-3-phosphate dehydrogenase | P17244 | intracellular | 2,14% | x | x | x | |
| | G3HMD1 | | | | | | |
| Actin beta | G3GVD0 | intracellular | 1,85% | x | x | x | |
| Sulfated glycoprotein 1 | G3I1Y9 | extracellular | 1,83% | x | x | x | |
| L-lactate dehydrogenase | G3I255 | intracellular | 1,55% | x | | | |
| Basement membrane-specific heparan sulfate proteoglycan core protein | G3HIM1 | plasma membrane | 1,54% | x | | | |
| | A0A3L7I8L8 | | | | | | |
| Phosphopyruvate hydratase | G3IAQ0 | intracellular | 1,46% | x | x | | |
| Lipoprotein lipase | G3H6V7 | extracellular | 1,31% | x | | | |
| | A0A3L7IKX6 | | | | | | |
| Endoplasmic reticulum chaperone BiP | G3I8R9 | intracellular | 1,25% | x | x | x | |
| Heat shock cognate 71 kDa protein | P19378 | intracellular | 1,24% | x | | | |
| Elongation factor 2 | G3HSL4 | intracellular | 1,16% | x | x | | |
| | P09445 | | | | | | |
| Cathepsin B | G3H0L9 | intracellular | 1,15% | x | x | x | |
| Heat shock protein HSP 90-alpha | P46633 | intracellular | 1,11% | x | | | |
| Glutathione S-transferase | G3I3Y6 | intracellular | 1,00% | x | x | x | |
| Elongation factor 1-alpha 1 | P62629 | intracellular | 0,96% | x | | | |
| | G3HH39 | | | | | | |
| Nidogen-1 | G3I3U5 | extracellular | 0,84% | x | | | |
| Ubiquitin | G3I129 | intracellular | 0,80% | x | x | x | x |
| Serine protease HTRA1 | G3IBF4 | extracellular | 0,78% | x | x | | |
| Carboxypeptidase | G3H8V5 | extracellular | 0,66% | x | x | x | |
| Peptidyl-prolyl cis-trans isomerase | G3HIQ1 | intracellular | 0,66% | x | | | |
| Endoplasmin | G3HQM6 | intracellular | 0,58% | x | x | | |
| Phosphoglycerate kinase 1 | P50310 | intracellular | 0,56% | x | | | |
| Histone H2B | G3INX0 | intracellular | 0,49% | x | | | |
| Tubulointerstitial nephritis antigen-like | G3H1W4 | intracellular | 0,47% | x | x | | |
| Annexin | G3IG05 | extracellular | 0,38% | x | x | x | x |
| Histone H2A type 1 | G3HDT6 | intracellular | 0,37% | x | | | |
| Calreticulin | G3HCX8 | intracellular | 0,36% | x | x | | |
| Galectin | G3I4Z7 | extracellular | 0,36% | x | | | |
| | A0A3L7I2M5 | | | | | | |
| Osteopontin | G3IKQ9 | extracellular | 0,27% | x | x | x | |
| 6-phosphogluconate dehydrogenase, decarboxylating | G3IHY5 | intracellular | 0,23% | x | | | |
| Protein S100 | G3HC31 | intracellular | 0,21% | x | x | | |
| 60S acidic ribosomal protein P0 | G3GU76 | intracellular | 0,21% | x | | | |
| | G3HKG9 | | | | | | |
| Malate dehydrogenase | G3HDQ2 | intracellular | 0,20% | x | | | |
| Granulins | G3HLK3 | extracellular | 0,20% | x | | | |
| Protein disulfide-isomerase | G3H0U6 | intracellular | 0,14% | x | | | |
| GTP-binding nuclear protein Ran | G3IHE5 | intracellular | 0,14% | x | | | |
| 14-3-3 protein theta | G3IEY9 | intracellular | 0,13% | x | | | |
| Hypoxia up-regulated protein 1 | G3I973 | intracellular | 0,06% | x | | | |
| Elongation factor 1-gamma | G3IE48 | intracellular | 0,02% | x | | | |
| Histone H2A | G3HDS3 | intracellular | <LLOQ | x | | | |
| Histone H4 | G3HPV6 | intracellular | <LLOQ | x | | | |
| 40S ribosomal protein SA | G3HQX0 | intracellular | <LLOQ | x | | | |
| Tubulin alpha chain | G3I6I6 | intracellular | <LLOQ | x | | | |
| Peptidyl-prolyl cis-trans isomerase A | G3IED5 | intracellular | <LLOQ | x | x | | |
| Tubulin beta chain | G3IEG6 | intracellular | <LLOQ | x | | | |

Of course, it is envisaged according to all aspects and embodiments of the present invention related to the modification of the expression of at least one ECM or ECM-related protein, preferably of a CHO cell, that these aspects and embodiments can be combined with the findings of the present inventors regarding acHCPs and/or drHCPs. This is particularly true in view of the fact that the two completely independent strategies of defining ECM and ECM-related proteins on the one hand side via transcriptome analysis, and the protein/peptide analysis for defining, grouping and stratifying acHCP/drHCP showed certain overlaps, i.e., candidates simultaneously qualifying as ECM(-related) and as acHCP and/or drHCPs. These targets thus represent particularly attractive candidates. Further, in preferred embodiments at least one ECM or ECM-related protein may represent the first modification target, whilst another acHCP and/or drHCP may represent another modification, preferably knock-down or knock-out, candidate to optimize the CHO cell expression pattern in a favorable way.

In preferred embodiments, the CHO cell according to the present disclosure may comprise at least one modification of a HCP that simultaneously qualifies as ECM/ECM-related protein and as acHCP and/or drHCP, alone, or in combination with at least one further modification. This has the great advantage that metabolic burden and HCP load of a final biotherapeutic product are simultaneously removed.

**Table 4** summarizes selected HCPs that were identified by the present inventors to qualify as ECM/ECM-related protein and as acHCP, whereas **Table 5** summarizes HCPs that simultaneously qualify as ECM/ECM-related protein and as drHCP. Those proteins highlighted in bold and underlined in **Table 5** qualify as ECM/ECM-related protein, as acHCP and as drHCP and thus represent highly interesting targets when generating CHO cells with a modified ECM profile.

**Table 4**

| **Protein name** | **Uniprot ID** | **Location** | **MRC** |
|---|---|---|---|
| Fibronectin | G3I1V3 | extracellular | 4.53% |
| | A0A3L7IDB0 | | |
| Basement membrane-specific heparan sulfate proteoglycan core protein | G3HIM1 | plasma membrane | 1.54% |
| | A0A3L7I8L8 | | |
| Lipoprotein lipase | G3H6V7 | extracellular | 1.31% |
| | A0A3L7IKX6 | | |
| Nidogen-1 | G3HWE4 | extracellular | 0.76% |
| | A0A3L7HVW3 | | |
| Peroxidasin | G3HBI1 | extracellular | 0.69% |
| | A0A3L7HCC3 | | |
| Biglycan | G3HSX8 | extracellular | 0.55% |
| | A0A061HUR7 | | |
| Galectin-3-binding protein | G3H3E4 | extracellular | 0.50% |
| Laminin subunit beta-1 | G3I278 | extracellular | 0.44% |
| | A0A3L7HMC9 | | |
| Galectin | G3I4Z7 | extracellular | 0.36% |
| | A0A3L7I2M5 | | |
| Glypican-1 | G3H4T5 | plasma membrane | 0.16% |
| | A0A3L7IDU1 | | |

**Table 5**

| **Protein name** | **Uniprot ID** | **Location** | **MRC** | **ProA** | **VI** | **CEX** | **AEX** |
|---|---|---|---|---|---|---|---|
| **Fibronectin** | **G3I1V3 A0A3L7IDB0** | extracellular | 4,53% | x | x | | |
| **Basement membrane-specific heparan sulfate proteoglycan core protein** | **G3HIM1 A0A3L7I8L8** | plasma membrane | 1,54% | x | | | |
| **Lipoprotein lipase** | **G3H6V7 A0A3L7IKX6** | extracellular | 1,31% | x | | | |
| Calreticulin | G3HCX8 | intracellular | 0,36% | x | x | | |
| **Galectin** | **G3I4Z7 A0A3L7I2M5** | extracellular | 0,36% | x | | | |

In a preferred embodiment of the CHO cell according to the present invention, the modified extracellular matrix (ECM) may be characterized by a reduced load of at least one endogenous protein being independently selected from the group consisting of proteins with an amino acid sequence according to any of the SEQ ID NOs: 2, 12, 29, 42, 54, 55, 77, 117, 108, 109, 119, 126, 124, 106, 123, 122, 107, 100, 102, 125, 105, 111, 90, 118, 91, 104, 113, 92, 112, 95, 114, 120, 93, 115, 98, 116, 103, 101, 110, 121, 99, 97, and 94 or with an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any of the amino acid sequences according to any of the SEQ ID NOs: 2, 12, 29, 42, 54, 55, 77, 117, 108, 109, 119, 126, 124, 106, 123, 122, 107, 100, 102, 125, 105, 111, 90, 118, 91, 104, 113, 92, 112, 95, 114, 120, 93, 115, 98, 116, 103, 101, 110, 121, 99, 97, and 94.

In all embodiments according to the various aspects and embodiments as disclosed herein, a CHO cell may comprise at least two or more modifications in two or more coding sequences coding for two or more endogenous ECM proteins and/or endogenous ECM-related proteins to reduce the metabolic burden as far as reasonably possible.

In certain embodiment, the at least one further modification of the at least two or more modifications is a modification of a sequence encoding an ECM protein or an ECM-related protein selected from the group summarized in Table 1, Table 4, Table 5, or Table 6 or any homologue, orthologue, or paralogue thereof, preferably wherein the at least one further modification of the at least two or more modifications is a modification of a sequence encoding an ECM protein or an ECM-related protein having an amino acid sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO2, 12, 14, 24, 28, 29, 36, 38, 42, 54, 55, 77, 94, 95, 96, 98, 101, 104, 106, 109, 110, 111, 112, 113, 124, 127 or 156, respectively.

**Table 6** summarizes a non-limiting list of HCPs currently characterized by the present inventors based on the methods as disclosed herein as ECM or ECM-related proteins. In certain embodiments, at least two, three, four, five, six, seven, eight, nine, ten, or even more ECM and ECM-related proteins are knocked-down or knocked-out simultaneously.

**Table 6**

| **No.** | **Protein name** | **Uniprot ID** | **SEQ ID NO:** |
|---|---|---|---|
| 1 | Fibronectin | G3I1V3 A0A3L7IDB0 | 2 |
| 2 | Basement membrane-specific heparan sulfate proteoglycan core protein | G3HIM1 A0A3L7I8L8 | 12 |
| 3 | Lipoprotein lipase | G3H6V7 A0A3L7IKX6 | 127 |
| 4 | Nidogen-1 | G3HWE4 A0A3L7HVW3 | 28, 156 |
| 5 | Peroxidasin | G3HBI1 A0A3L7HCC3 | 29 |
| 6 | Biglycan | G3HSX8 A0A061HUR7 | 128 |
| 7 | Galectin-3-binding protein | G3H3E4 | 38 |
| 8 | Laminin subunit beta-1 | G3I278 A0A3L7HMC9 | 42 |
| 9 | Calreticulin | G3HCX8 | 54 |
| 10 | Galectin | G3I4Z7 A0A3L7I2M5 | 55 |
| 11 | Glypican-1 | G3H4T5 A0A3L7IDU1 | 77 |
| 12 | Cyclic AMP-dependent transcription factor ATF-4 | G3IHE0 | 117 |
| 13 | A disintegrin and metalloproteinase with thrombospondin motifs 1 | A0A8C2MMI8 | 108 |
| 14 | A disintegrin and metalloproteinase with thrombospondin motifs 7 | A0A8C2M328 | 109 |
| 15 | Calreticulin | Q8K3H7 | 119 |
| 16 | Calreticulin-3 | G3H4H3 | 126 |
| 17 | Collagen alpha-2(V) chain | A0A8C2M1I7 | 124 |
| 18 | Collagen alpha-1(VII) chain | A0A8C2QLM9 | 106 |
| 19 | Catenin alpha-1 | G3HEY9 | 123 |
| 20 | Alpha-catulin | A0A8C2M0N5 | 122 |
| 21 | Catenin beta-1 | G3H996 | 107 |
| 22 | Beta-catenin-like protein 1 | G3GXY1 | 100 |
| 23 | Catenin delta-1 | A0A8C2LRW3 | 102 |
| 24 | Protein diaphanous homolog 1 | A0A8C2MW41 | 125 |
| 25 | Cytoplasmic dynein 1 heavy chain 1 | A0A8C2QIZ6 | 105 |
| 26 | EFEMP2 | O55058 | 111 |
| 27 | Eukaryotic translation initiation factor 3 subunit B | A0A3L7HQ14 | 90 |
| 28 | Eukaryotic translation initiation factor 3 subunit C | G3IK13 | 118 |
| 29 | FAD synthase | G3HC64 | 91 |
| 30 | Laminin subunit gamma-1 | A0A8C2LYC0 | 104 |
| 31 | Latent-transforming growth factor beta-binding protein 3 | A0A8C2MY83 | 113 |
| 32 | Leucine zipper protein 1 | A0A3L7I8J7 | 92 |
| 33 | Lactadherin | A0A8C2LVZ9 | 112 |
| 34 | Matrix metalloproteinase-28 | G3I8B5 | 95 |
| 35 | Osteoclast-stimulating factor 1 | G3ICW1 | 114 |
| 36 | RPSA | P38982 | 120 |
| 37 | Serpin H1 | G3IDD4 | 93 |
| 38 | Splicing factor 3B subunit 3 | G3HAF4 | 115 |
| 39 | SPARC (Osteonectin) | A0A8C2LJ19 | 96 |
| 40 | Transforming growth factor beta-1 proprotein | A0A8C2MC57 | 98 |
| 41 | Transforming growth factor beta-1-induced transcript 1 protein | A0A8C2M0V0 | 116 |
| 42 | Transforming growth factor beta-3 proprotein | G3HFS5 | 103 |
| 43 | Protein-glutamine gamma-glutamyltransferase 2 | G3GXZ0 | 101 |
| 44 | Trinucleotide repeat-containing gene 6A protein | A0A8C2M6G9 | 110 |
| 45 | Trinucleotide repeat-containing gene 6B protein | G3HW00 | 121 |
| 46 | Trinucleotide repeat-containing gene 6C protein | A0A8C2N0F8 | 99 |
| 47 | Thioredoxin domain-containing protein 5 | A0A8C2N4D5 | 97 |
| 48 | Protein Wnt-4 | A0A3L7I8K8 | 94 |

In a further embodiment according to the various aspects as disclosed herein, there is provided a CHO wherein the at least one further modification is a modification of a sequence encoding an ECM protein or an ECM-related protein selected from the group summarized in Table 1, Table 4, Table 5, or Table 6, or any homologue, orthologue, or paralogue thereof and/or wherein the at least one further modification is a modification of a sequence encoding an abundant core HCP (acHCP) and/or a difficult to remove HCP (drHCP) selected from the group summarized in Table 2 and Table 3 respectively, or any homologue, orthologue, or paralogue thereof.

In another embodiment of the various aspects as disclosed herein, the CHO cell may comprise at least two or more modifications in two or more coding sequences coding for two or more endogenous ECM proteins and/or endogenous ECM-related proteins, wherein at least one of the at least two or more modifications is in a sequence encoding an ECM protein or an ECM-related protein being selected from the group consisting of Fibronectin, Basement membrane-specific heparan sulfate proteoglycan core protein, Peroxidasin, Biglycan, Galectin-3 binding protein, Laminin subunit beta-1, Calreticulin, Galectin, Glypican, or any homologue, orthologue, or paralogue thereof; more preferably wherein at least one of the at least two or more modifications is in a sequence encoding an ECM protein or an ECM-related protein having an amino acid sequence corresponding to SEQ ID NO: 2, 12, 29, 36, 38, 54, 55, 77, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: SEQ ID NO: 2, 12, 29, 36, 38, 54, 55, 77, respectively.

In a particularly preferred embodiment of the CHO cell according to the present invention, the modified extracellular matrix (ECM) may be characterized by a reduced load of at least Fibronectin according to SEQ ID NO: 2, or Uniprot ID G3I1V3, or any homologue, orthologue, or paralogue thereof, or by a reduced load of a protein having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to SEQ ID NO: 2.

Preferably, at least one ECM protein or an ECM-related protein modified according to the present disclosure may simultaneously be an abundant core HCP (acHCP) and/or a difficult to remove HCP (dcHCP). This has the huge advantage that a rather huge percentage of HCPs can be removed significantly facilitating the purification scheme and thus reducing costs whilst maintaining the overall bioprocess performance. For example, the ECM protein fibronectin (extracellular; G3I1V3 or A0A3L7IDB0) is simultaneously an acHCP and a dcHCP having a mean relative concentration of 4.53% (the mean relative concentration having been determined as detailed in the Examples), Basement membrane-specific heparan sulfate proteoglycan core protein Hspg2 (plasma membrane, G3HIM1 or A0A3L7I8L8) has a mean relative concentration of 1.54%. Lipoprotein lipase (G3H6V7 or A0A3L7IKX6, Lpl, extracellular) has a mean relative concentration of 1.31%, Nidogen-1 (G3I3U5, G3HWE4 or A0A3L7HVW3, Nid1, extracellular) has a mean relative concentration of 0.76%, Peroxidasin (G3HBI1 or A0A3L7HCC3, Pxdn, extracellular) has a mean relative concentration of 0.69%, Biglycan (G3HSX8 or A0A061HUR7, Bgn, extracellular) has a mean relative concentration of 0.55%, Galectin-3-binding protein (G3H3E4, Lgals3bp, extracellular) has a mean relative concentration of 0.50%, Laminin subunit beta-1 (G3I278 or A0A3L7HMC9, Lamb1, extracellular) has a mean relative concentration of 0.44%, Calreticulin (G3HCX8, Calr, intracellular) has a mean relative concentration of 0.36%, Galectin (G3I4Z7 or A0A3L7I2M5, Lgals1, extracellular) 0.36%, and Glypican-1 (G3H4T5 or A0A3L7IDU1, Gpc1, plasma membrane) has a mean relative concentration of 0.16%. Removing, for example, at least FN, Hspg2 and/or LPL can thus be preferred to eliminate a huge amount of HCPs representing acHCPs. FN, Hspg2, LPL, Calr and Lgals1 also represent drHCPs. Removing at least one of these, or all, ECM or ECM-related proteins simultaneously can thus have a significant advantage on DSP processes.

In certain embodiments of all aspects as disclosed herein, the at least one modification leads to the reduced transcription and/or reduced functional expression of said endogenous ECM protein(s) and/or ECM-related protein(s) thereby lowering the total content of endogenous ECM proteins and/or endogenous ECM-related proteins. This early effect results in a reduced transcriptional and thus metabolic burden so that the cell can invest the metabolic energy in expressing other effectors, including a therapeutic protein of interest.

Preferably, the CHO cell according to the present various aspects and embodiments as disclosed herein may three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, or twenty or more modifications in three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, or twenty or more coding sequences, respectively, wherein each of said modifications affects a protein being classified as an endogenous ECM protein or an endogenous ECM-related protein, preferably wherein at least one ECM protein or an endogenous ECM-related protein is independently selected from SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, respectively.

In yet another embodiment of the various aspects as disclosed herein, the CHO may comprise at least one recombinant gene encoding at least one recombinant protein of interest and/or encoding at least one recombinant RNA molecule of interest, preferably wherein said at least one recombinant protein of interest is a therapeutic molecule.

The term "recombinant" as used herein, and as generally defined above, usually refers to molecules, e.g. proteins or nucleic acid molecules, which are non-endogenous to the cells, in which they are expressed. For example, genetic material could have been introduced into a cell, which afterwards can express (transcribe and/or translate) the recombinant molecule(s) of interest.

In certain embodiments, it may be preferable to modify more than one ECM or ECM-related protein, optionally even in combination with an additional ECM or ECM-related protein and/or a further acHCP and/or drHCP in one and the same setting in a multiplexing approach, particularly for embodiments, where at least one recombinant molecule of interest is expressed, as it is very preferred that the CHO cell shifts all its metabolic capacity to the production of the desired recombinant molecule. First, the strategy of the present invention allows to reduce the metabolic burden of the cell so that the cellular machinery can focus on the production of the recombinant protein of interest. Secondly, this may allow for the reliable elimination of certain abundant and difficult-to-remove HCPs in the final biopharmaceutical independent of any upstream processing methods or downstream processing methods whilst simultaneously maintain a high performance rate of the CHO cell regarding its growth and titer capacity to produce high yields of a functional therapeutic agent of interest. Thirdly, performing the knock-out in one and the same assay reduces the number of iterative modifications to a host cell to be modified, when multiple HCPs are targeted in one and the same experiment. In general, all target HCPs specified in any one of Tables 1 to 5 above, alone or in combination, qualify as suitable knock-out target. Finally, as reported for certain ECM and ECM-related proteins herein, the elimination or reduction thereof by modification does not only allow the reduction of the metabolic burden (transcription level) and/orthe overall HCP load (expression level). Surprisingly, the present inventors could identify certain targets, including FN, that are completely dispensable for cellular integrity and performance during recombinant protein production and even lead to an improved bioprocess parameter, including, but not being limited to peak VCC, specific productivity, final titer, process duration, etc.

In a specific embodiments, at least one, at least 2, at least three, at least four, at least five, at least sic, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty seven, at least twenty-eight, at least twenty-nine, at least thirty, or more, or all HCP targets according to Table 1, 2, or 3 above may be simultaneously knocked-out to obtain a modified cell according to the present disclosure. This is due to the fact that it could be shown experimentally that the impact of single knock-outs of exemplary HCPs taken from Table 1 and Table 2 on growth/viability and productivity/titer of the engineered cells has been evaluated under bioprocess conditions by generating stable mAb expressing cell clones followed by fed-batch runs in shake flask and was not negative so that the targets are suitable for knock-outs alone or in combination.

In preferred embodiments of combined knock-outs, at least one of the knock-out targets is Fibronectin (Uniprot ID: G3I1V3; SEQ ID NO: 2), or any homologue, orthologue, or paralogue thereof.

In one embodiment of all aspects and embodiments as disclosed herein, the modified CHO cell may be selected from, or may be derived from, meaning originating from, a CHO cell variant selected from the group consisting of CHO-K1, CHO-DXB11 (synonymously referred to as CHO-DUKX), CHO-DG44, CHO-S, and CHO-Pro minus, or a CHO GS knock out. Alternatively, the modified CHO cell according to the present invention may also be derived from other CHO cell variants.

In a preferred embodiment, the modified CHO cell may be, or may be derived from a CHO-DG44.

In another preferred embodiment, the modified CHO cell may be, or may be derived from a CHO-K1.

In yet a preferred, the modified CHO cell may be, or may be derived from CHO-DXB11 (synonymously referred to as CHO-DUKX).

In yet another preferred embodiment, the CHO cell may be, or may be derived from a CHO GS knock out cell.

In one preferred embodiment, the modified CHO cell may be derived from CHO-S, or it may be derived from CHO-Pro minus.

In a preferred embodiment, the modified CHO cell according to the present invention may contain one or more exogenously added gene(s) coding for one or more recombinant molecules, including at least one recombinant protein or peptide, RNA or DNA of interest. Preferably, said one or more recombinant molecule is a recombinant protein or peptide of interest representing a biopharmaceutical protein being selected from the exemplary group consisting of a recombinant protein or peptide, including monoclonal antibodies or a single-chain Fv, or any other antibody-format, including mono-, bi- and multispecific antibodies, chimeric antigen receptors, virus-like proteins, vaccines, and immunogenic compounds of any kind.

In one embodiment of the present invention, the modified CHO cell may be derived from CHO-DG44 and the at least one modification may be present in at least one endogenous coding sequence, which codes for a protein selected from group consisting of Thrombospondin-1 (Uniprot ID: G3HHV4; SEQ ID NO: 1), Fibronectin (Uniprot ID: G3I1V3; SEQ ID NO: 2), Pyruvate kinase (Uniprot ID: G3H3Q1 ; SEQ ID NO: 4) as the at least one ECM or ECM-related and/or acHCP and/or drHCP protein as disclosed herein, or in addition to at least one ECM or ECM-related protein as disclosed herein, preferably wherein the modified CHO cell may contain one exogenously added gene coding for a recombinant protein of interest, wherein said recombinant protein of interest is a monoclonal antibody, or a fragment or domain thereof, or any further therapeutic protein, including a bispecific antibody, fusion proteins, peptibodies, and peptides, or any prophylactic agent, including a vaccine, or a fragment or portion thereof.

In one embodiment, at least one of the protein to be knocked-out is selected from at least one of, or a specific combination of a knock-out of the HCP(s) FN, LPL, THBS, BGN, NID-1, PCOLCE, PXDN, THBS, PKM, CSPG4, LDHA, VIM, HSPA5, PPIB, HSP90AA1, LAMA5, FSTL1, and/or AEBP1 or any ortholog, homolog or paralog thereof, and/or additionally including at least one of PSAP, PRDX HSPG2, CTSB, LGALS3BP, TNX, LAMB1, ITIH5, or TIMP as the at least one ECM or ECM-related protein as disclosed herein, or in addition to at least one ECM or ECM-related protein as disclosed herein.

In a preferred embodiment of all aspects and embodiments as disclosed herein, at least one of FN, LPL, THBS, BGN, NID-1 , PCOLCE, PXDN, THBS, PKM, CSPG4, LDHA, VIM, HSPA5, PPIB, HSP90AA1, LAMA5, FSTL1, AEBP1, PSAP, PRDX HSPG2, CTSB, LGALS3BP, TNX, LAMB1, ITIH5, and/or TIMP, or any ortholog, homolog or paralog thereof is knocked-out as the at least one ECM or ECM-related protein as disclosed herein, or in addition to at least one ECM or ECM-related protein as disclosed herein.

In one embodiment of the present invention, the at least one modification may lead to premature transcription termination resulting in a truncated and thus inactive form of the respective protein. Preferably, the at least one modification may lead to premature transcription termination in exon 1 of the respective protein. In another preferred embodiment, the mutation may be in another exon than exon 3, e.g., in case another exon is easier accessible for successful and efficient editing.

Preferably, premature transcription termination may occur in the first 50%, preferably in the first 40%, preferably in the first 30%, preferably in the first 20%, preferably in the first 10%, preferably in the first 5%, preferably in the first 2% of the respective genomic coding sequence viewed from its transcriptional start, i.e. its start codon.

By prematurely terminating the transcription of a given target gene as close to its respective transcriptional start as possible the amount of formed transcript is kept to a minimum and the overall load of the respective protein in the modified CHO cell according to the present invention is minimized. Thereby, the positive effect in terms of an optimized downstream processing efficiency is maximized.

Preferably, the CHO cell modified according to the present invention comprises at least one recombinant gene encoding at least one recombinant protein of interest and/or encoding at least one recombinant RNA molecule of interest.

In a preferred embodiment of all aspects and embodiments as disclosed herein, the at least one recombinant protein of interest may be selected from the group consisting of therapeutic proteins, monoclonal antibodies, bispecific antibodies, fusion proteins, peptibodies, and peptides.

In one embodiment of all aspects and embodiments as disclosed herein, the at least one recombinant RNA molecule of interest may be selected from the group consisting of mRNA therapeutics including mRNA vaccines, non-coding single-stranded RNA species, such as e.g. antisense RNAs and MicroRNAs (also called miRs or miRNAs), interfering RNAs (RNAi), such as e.g. small interfering RNA (siRNA) or micro RNA (miRNA), and RNA aptamers.

In a preferred embodiment of the CHO cell according to the present invention, the at least one recombinant protein of interest is a therapeutic molecule.

In certain embodiments of the various aspects as disclosed herein, the CHO cell comprises at least one modification, which may be selected from the group consisting of at least one insertion, at least one deletions, and at least one substitution, including a base edit, or any combination thereof, preferably wherein said at least one modification is present in exon 1 of the respective endogenous coding sequence, and/or wherein said at least one modification in said coding sequence is a frame-shift mutation or a point mutation, the point mutation resulting in a stop codon.

In a preferred embodiment of the present invention, the at least one modification in said endogenous coding sequence(s) may allow for an optimized downstream processing, wherein said optimized downstream processing is characterized by a reduced number of downstream processing steps required for obtaining a recombinantly produced molecule in its purified form as compared to the respective wild-type (i.e non-modified) CHO cell. For example, the at least one modification may have the advantage that at least one purification step, particularly an ion exchange chromatography step, i.e., anion or cation exchange chromatography, or even both, can be omitted, as the overall HCP load was increased that significantly by the methods of the present invention that these steps are no longer necessary to achieve the ultimate grade of purity. This represents a significant advantage, as additional column chromatography steps require resources and each additional step leads to a decrease of the yield of a recombinant protein of interest to be purified.

Thus, the modified CHO cell according to the present invention allows for the downstream processing to be optimized in terms of cost efficiency, time efficiency, energy efficiency, and sustainability.

In certain embodiments of the aspects as disclosed herein, the CHO cell may have at least one, wherein said at least one modification in said at least one endogenous coding sequence allows for an improved bioprocess performance, wherein said improved bioprocess performance is characterized by an increased concentration of viable cells and/or an increased specific productivity and/or an increased final titer and/or an increased process duration in comparison to a wild-type cell not carrying the at least one modification in its genome. As detailed in the Examples Section, the aforementioned exemplary bioprocess performance characteristics are relevant indicators for the viability of a cell and the productivity thereof in recombinant expression technologies. The skilled person is aware of these bioprocess performance parameters and can easily determine the same, and additional (key) performance and quality indicators for a given cellular / assay system of interest.

In certain embodiments of all aspects disclosed herein, said at least one modification in said endogenous coding sequence may be a frame-shift mutation or a point mutation, the point mutation resulting in a stop codon.

In yet another embodiment of all aspects disclosed herein, all alleles of said at least one endogenous coding sequence may comprise at least one or more of said modification(s).

In a second aspect, there is provided a method for the production of at least one modified CHO cell according to the first aspect above, wherein the method may comprise the steps of: (i) providing at least one CHO cell comprising at least one endogenous target nucleic acid segment; (ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

Biotechnological engineering of CHO cells started by employing conventional homologous recombination-based gene targeting strategies (Yamane-Ohnuki et al., 2004, doi: 10.1002/bit.20151). However, such strategies proved rather ineffective as homologous recombination (HR) occurs several orders of magnitude less frequently as compared to non-homologous end-joining (NHEJ) in mammalian cells (Sedivy and Sharp, 1989, doi: 10.1073/pnas.86.1.227). NHEJ is particularly applicable for generating gene disruptions, as it is an imperfect repair process often inducing insertions or deletions at the site of the double-strand break (DSB). However, for generating gene disruptions in a site-specific manner, endonucleases suitable for sequence-specific targeting are required, such as transcription activator-like effector nucleases (TALENs), zinc-finger nucleases (ZNFs), CRISPR/Cas effectors, and meganucleases, which were all successfully applied to mammalian cell, i.e. human or CHO cells (Galetto et al., 2009 DOl: 10.1517/14712590903213669; Santiago et al., 2008 doi.org/10.1073/pnas.0800940105; Miller et al., 2010 DOI: 10.1038/nbt.1755; Hillary and Ceasar,. Mol Biotechnol 65, 311-325 (2023). https://doi.orq/10.1007/s12033-022-00567-0).

In yet another aspect, there is provided a method for the production of at least one recombinant molecule, preferably at least one recombinant protein, of interest comprising the steps: (a) providing at least one modified CHO cell of the second aspect, wherein the at least one modified CHO cell comprises at least one recombinant gene encoding at least one recombinant protein, DNA or RNA of interest; (b) culturing said at least one target cell in a culture medium such that at least one recombinant molecule of interest is transcribed and/or translated; (c) harvesting said at least one recombinant molecule of interest; (d) purifying and/or decontaminating said at least one recombinant molecule, preferably the at least one recombinant protein of interest.

In yet another aspect, there is provided a use of at least one modified CHO cell according to any embodiment of the first aspect of the present invention for the production of at least one pharmaceutical drug.

In yet a further aspect, there is provided a kit comprising (a) at least one modified CHO cell according to any embodiment of the first aspect of the present invention, the CHO cell or the kit comprising: (b.i) at least one nucleic acid molecule suitable for expressing at least one recombinant molecule of interest, preferably at least one recombinant protein of interest; optionally means for introducing the same into the CHO cell genome, or alternatively (b.ii) at least one nucleic acid molecule encoding at least one recombinant molecule of interest, preferably at least one recombinant protein of interest; optionally means for introducing the same into the CHO cell genome; or alternatively (b.iii) at least one nucleic acid molecule suitable for transcribing at least one recombinant RNA molecule of interest; optionally means for introducing the same into the CHO cell genome; or alternatively (b.iv) at least one nucleic acid molecule encoding at least one recombinant RNA molecule of interest; optionally means for introducing the same into the CHO cell genome; and optionally (c) culture medium suitable for the at least one modified CHO cell according to step (a) (i) to replicate the at least one nucleic acid molecule according to any of the steps (b.i), (b.ii), (b.iii), and (b.iv) endogenously or exogenously; and (ii.a) to express the at least one recombinant protein of interest according to step (b.i) and/or (b.ii), and/or to transcribe the at least one recombinant RNA molecule of interest according to step (b.iii) and/or (b.iv).

In certain embodiments related to the CHO cell according to the present invention, or methods, kits and uses, including medical uses, associated therewith all alleles of said at least one endogenous coding sequence comprise at least one or more of said modification(s). In certain embodiments, only one allele may be targeted to achieve a favourable balance result between the reduction of the load of the respective HCP to be knocked-out in one allele and host cell integrity and stability in cases where a full knock-out might be detrimental to the cell, or might be at least detrimental in a sense that the cell growth, and recombinant protein production capacity is significantly reduced.

In a preferred embodiment of the various method according to the present invention, the at least one endogenous target nucleic acid segment may be a nucleic acid sequence, preferably a DNA sequence, coding for part of any of the amino acid sequences according to any of the SEQ ID NOs and/or HCP target according to any one of Tables 1, 2, 3, 4, 5, or 6.

Preferably, said target nucleic acid segment may be a nucleic acid sequence, preferably a DNA sequence, coding for an amino acid sequence, which is contained in the first 50%, preferably in the first 40%, preferably in the first 30%, preferably in the first 20%, preferably in the first 10%, preferably in the first 5%, preferably in the first 2% of any of the amino acid sequences according to any of the SEQ ID NOs and/or HCP target according to any one of Tables 1, 2, 3, 4, 5, or 6.

In a preferred embodiment of the methods for production of at least one modification in a cell, preferably a CHO cell, according to the present invention, a CRISPR/Cas system may be used as genome editing system. This clustered regularly interspaced short palindromic repeats (CRISPR) and CRISPR-associated protein (CRISPR/Cas) system has significantly altered molecular biology research and development over the last decade offering enormous possibilities to modify cells of interest, including complex eukaryotic cells, in a highly targeted way. Any CRISPR/Cas system suitable for eukaryotic cell genome editing can be used according to the present disclosure. The CRISPR/Cas system may include a CRISPR/Cas9 systems, a CRISPR/Cas13 systems, CRISPR/Cas12a system (also called CRISPR/Cas12a system), a CRISPR/C2C2 systems, CRISPR/CasX systems (also called CRISPR/Cas12e system), CRISPR/CasY systems, CRISPR/Cmr systems, CRISPR/Csm systems, CRISPR/MAD2 systems, CRISPR/MAD7 systems, CRISPR/CasZ systems, CRISPR/Cas14a, b or c systems, CRISPR/Cas phi systems, CRISPR/Cas12f systems, or catalytically active fragments or variants thereof. Various kinds of CRISPR systems and optimized mutated systems, including nickase or nuclease-dead based systems, CRISPR mutants with optimized PAM-specificities and temperature tolerance, and various methods of using these CRISPR variants and mutants alone or on combination with other effectors, e.g., to be suitable for base editing and prime editing, have been developed and are suitable to be used according the present disclosure (cf. Jinek, et al., A Programmable Dual-RNA-Guided DNA Endonuclease in Adaptive Bacterial Immunity. Science 2012, 337, 816-821; Hillary and Ceasar, Mol Biotechnol. 2022 Sep 27;1-15, doi: 10.1007/s12033-022-00567-0; Zetsche, et al. Cpf1 Is a Single RNA-Guided Endonuclease of a Class 2 CRISPR-Cas System. Cell 2015, 163, 759-771; Kim et al. Nat Biotechnol. (2022);40(1):94-102; doi: 10.1038/s41587-021-01009-z; Liu et al., CRISPR J. April 2020; 3(2): 97-108.; Karvalis et al. Nucleic Acids Res. (2020); 48(9):5016-5023. doi: 10.1093/nar/gkaa208, Selkova et al. RNA Biol. (2020); 17(10):1472-1479; doi: 10.1080/15476286.2020.1777378, Rees and Liu, 2018. Nat. Rev. Genet.; doi: 10.1038/s41576-018-0059-1, Komor et al., Nature 533, 420-460, 2016; Komor et al., 2017, Science Advances, doi:10.1126/sciadv.aao4774M; Gaudelli et al., Nature, 551: 464-471, 2017; Lu et al., nt. J. Mol. Sci. 2022, 23, 9862. https://doi.org/10.3390/ijms23179862).

In certain embodiments, CRISPR nucleases or variants thereof being suitable for multiplexing applications, meaning the targeting of more than one target site of interest in a genome to be modified in a site directed manner, may be preferred. CRISPR/Cas nucleases, or mutants or variants thereof, of the Class 2 Type V may be preferred in certain embodiments (cf. Koonin & Makarova. Mobile genetic elements and evolution of CRISPR-Cas systems: All the way there and back. Genome Biology and Evolution. 2017;9(10):2812-2825. doi: 10.1093/gbe/evx192, Tong et al., Front. Cell Dev. Biol 2021, Sec. Cellular Biochemistry doi.org/10.3389/fcell.2020.622103.

As it is known to one skilled in the art, CRISPR/Cas effectors need a guiding molecule to exert their activity. The "guide molecule", in particular the "guide RNA" (gRNA) may comprise a trans-activating CRISPR RNA (tracrRNA) and a CRISPR RNA (crRNA). Certain CRISPR nucleases, e.g., Cas12a, only require a crRNA. TracrRNA and crRNA can be combined in one molecule, resulting in a single guide RNA (sgRNA), which comprises the sequence information targeting the genomic sequence for cleavage by the nuclease. For multiplexing purposes, more than one gRNA allowing the recognition and thus modification of more than one target site in a genome may be designed and used in one and the same experiment, or one after another.

In a preferred embodiment of the method according to the present invention, the CRISPR-nuclease may be a Class 2 Type V nuclease, or a variant or mutant thereof, including a Cas12a (formerly known as Cpf1) or a MAD7 or a Cas14 effector. Suitable vectors and plasmids to express the relevant CRISPR effector and the cognate gRNA(s) are available to the skilled person and can be easily adapted by designing the gRNA(s) *in silico.*

In another embodiment, an RNAi agent can be used to knock-down or silence at least one RNA coding for at least one HCP target of interest. For RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". With these RNA hairpin sequences, more than one HCP target can be knocked-down simultaneously in certain embodiments.

In specific embodiments, a knock-down of at least one HCP via, for example, an RNAi agent and a knock-out mediated by genome editing, including CRISPR/Cas, may be combined to reduce the expression of at least one target HCP.

In an even further aspect of the invention, a method for the production of at least one recombinant protein of interest is provided comprising the steps: (a) providing at least one modified CHO cell according to the present invention, wherein the at least one modified CHO cell comprises at least one recombinant gene encoding at least one recombinant protein of interest; (b) culturing said at least one target cell in a culture medium such that at least one recombinant protein of interest is expressed; (c) harvesting said at least one recombinant protein of interest; (d) purifying and/ or decontaminating said at least one recombinant protein of interest.

In a preferred embodiment of the method according to the present invention, the at least one recombinant gene is exogenously expressed from at least one expression vector, i.e. at least one plasmid. Said expression vector may be particularly selected from the group consisting of mammalian expression vectors, as these are disclosed herein, or as these are known to one skilled in the art. The skilled person can thus easily define suitable expression vectors for a host cell of interest in his/her respective technical field that are compatible with the present disclosure.

In a preferred embodiment of the method according to the present invention, culturing said at least one target cell may be performed by a method selected from the group consisting of batch cultivation, fed-batch cultivation, perfusion cultivation, and the like, and combinations thereof. In a preferred embodiment of the method according to the present invention, the culture medium for culturing said at least one target cell may be selected from the group consisting of, for example, a non-chemically defined or a chemically-defined cell culture medium.

In one embodiment of the method according to the present invention, purifying and/or decontaminating may comprise one or more process steps selected from the group consisting of affinity purification, ion exchange, hydrophobic interaction chromatography, and size exclusion chromatography.

In a preferred embodiment of the method according to the present invention, the at least one recombinant protein of interest may be selected from the group consisting of, but not restricted to, therapeutic proteins, monoclonal antibodies, bispecific antibodies, fusion proteins, peptibodies, and peptides.

In a further aspect of the present invention, the use of at least one modified CHO cell according to any of the present invention for the production of at least one pharmaceutical drug, or a part thereof is provided. The pharmaceutical drug can be any biomolecule, preferably a recombinant molecule, including recombinant proteins, peptide, RNA, DNA, or any combination, fusion molecules, including covalently or non-covalently (e.g., hybridized; antigen::antibody complexes etc.) tethered molecules, that can be produced in a CHO cell of the present disclosure. The pharmaceutical drug will preferably be purified. Further, the pharmaceutical drug may be further modified, e.g., by chemical synthesis to attach an additional moiety, or to link two molecules to each other. Further, the pharmaceutical drug may be stabilized, lyophilized, and provided together with suitable buffers and agents all being pharmaceutically acceptable. Galenic techniques for compounding a pharmaceutical drug are known to one skilled in the art.

Further provided are methods for using the purified recombinant molecules, alone or in combination with further pharmaceutically acceptable ingredients and buffers, as produced by the modified CHO cells of the present invention in a method of treating a subject in need thereof. In view of the high degree of purity based on the reduction of the HCP amount, the recombinant molecules are particularly suitable to be safely applied to human beings.

In yet another aspect of the present disclosure, there is provided a method for identifying and optionally selecting a favourably modified host cell protein (HCP) profile of cell, preferably of a CHO cell, preferably a CHO cell characterized by a defined load of at least one endogenous protein being an ECM or an ECM-related protein and/or a difficult-to-remove HCP (drHCP) and/or an abundant core HCP (acHCP), wherein said method comprises: (i) providing at least one CHO cell, optionally a CHO cell expressing at least one recombinant molecule of interest; (ii) providing at least two, preferably at least 3, 4, 5, 6, 7, 8, 9, 10, or even more expression profiles for individual clones of at least one CHO cell expressing the same or a different recombinant molecule of interest; (iii) comparing and thus evaluating the data obtained from the expression profile analysis and correlating the data to at least one quantifiable parameter for each protein or RNA of interest; (iv) categorizing the data by attributing the values obtained for the at least one quantifiable parameter to at least one state or group; and (v) identifying favourably modified host cell protein (HCP) profile; the method optionally including preparing a cell, preferably a CHO cell, with a favourably modified HCP profile by modifying at least one endogenous HCP and optionally selecting and thus obtaining a modified cell, preferably a modified CHO cell, with a favourably modified HCP profile.

Correlation can be performed by in silico analytic methods. Usually, correlation will imply the alignment of the obtained data with publicly available genome, transcriptome and protein expression databases.

In one embodiment, the expression profile may be a transcriptome profile determining and/or quantifying RNA transcripts. In another embodiment, the expression profile may be a profile determining and/or quantifying a protein or peptide profile, preferably a high-throughput method including LC-MS and SWATH LC-MS.

In certain embodiments, the expression profile may be obtained by making a series of sample measurements over time, e.g., by taking and analysing a sample at certain time points before harvest, or by taking and analysing a sample after a post-harvest purification step, or by taking and analysing a sample, e.g., for a transcriptome profile, after stimulating a culture comprising the at least one CHO cell of interest, or a population thereof, with at least one stimulus or inducer to evaluate the influence of the stimulus or inducer (e.g., a chemical agent, a biological agent or any abiotic stress factor) on the cell or cell population.

Modification of the at least one cell, preferably the at least one CHO cell, can be performed as defined herein for the various methods for providing a CHO cell with a modified HCP profile.

The above methods thus integrate and facilitate high-throughput cell cultivation, screening and modification methodologies to expedite HCP target identification and to establish a pipeline for rapidly identifying HCP targets of interest for a setting of interest followed by a quick modification of the cell of interest, preferably in a multiplexing manner, to obtain optimized production CHO host cells in a short period of time.

In another embodiment there is provided a recombinant molecule as pharmaceutical agent, preferably a recombinant protein or peptide, including at least one monoclonal antibody, obtained from a modified CHO cell according to the present invention for use in a method of treating a subject in need thereof, including preventing a disease and curing a disease, wherein the treatment is associated with less side effects in view of the reduced HCP amount of the recombinant molecule as active pharmaceutical agent.

All individual embodiments and aspects as disclosed herein can be combined with each other within the framework and context of the present disclosure.

The present invention is now further illustrated by the following non-limiting examples.

### Examples

### Example 1: Identification of ECM and ECM-related proteins

### Example 1.1: Strategy and basis for identification

As a basis for the identification of relevant ECM or ECM-related knock-out targets, a number of mAb production clones was processed under industrial USP (Ambr^{®} 250 fed-batch process) conditions as listed in Tab. 1. Cell samples were taken daily throughout the whole process starting on either day 1 (CRG13) or day 3 (CRG14). Up to three samples per day were taken for CRG13 and one per day for CRG14 resulting in 192 and 125 samples, respectively (cf. **Fig. 1A**).

RNA was extracted and sequenced as 150 bp paired-end reads with the Illumina NovaSeq 6000 platform by Eurofins Genomics Germany GmbH (Ebersberg, Germany).

### Example 1.2: Bioinformatics

Raw reads were processed by the in-house RNA-seq analysis pipeline to obtain gene expression counts. In brief, raw sequences were quality controlled with fastQC v0.11.9 (Babraham Bioinformatics 2010) and multiQCv1.12 (Ewels et al. 2016), adapter and quality trimmed with Trimmomatic v0.39 (Bolger, Lohse, and Usadel 2014) and pseudo-aligned to our in-house genome assembly with kallisto v0.48.0G (Bray et al. 2016) to generate transcriptome profiles.

In detail, the following procedure was applied (which is also summarized in **Fig. 1B**):
1) Two reference data sets for ECM and ECM-related proteins were generated based on information available in public databases: a) MatrixDB, The Extracellular Matrix Interaction Database (Clerc et al. 2019), specifically 'Extracellular matrix proteins' from the MatrixDB biomolecules section (http://matrixdb.univ-lyon1.fr/download//proteins ECM.csv, last retrieved 2022-09-30); and b) UniProt Knowledgebase (The UniProt Consortium 2021) searched for taxonomy 'Rodentia' + Keyword 'Extracellular matrix' ((taxonomy_id:9989) AND (keyword:KW-0272), last searched 2022-09-30).
2) All genes that are listed in one or both reference data sets were identified in our in-house CHO genome assembly based on identifier mapping using custom bash commands. Only genes for which identifiers could be unambiguously mapped were kept for further analysis resulting in two gene sets: a) the 'union' gene set, comprising all annotated genes listed either in the one, the other or both reference datasets (union function in R). This set contained more genes than the 'intersection' set but genes might have only been validated by one of the two public databases and b) the 'intersection' gene set, comprising all annotated genes listed in both reference data sets (intersect function in R). This set contained less genes and represented a more conservative approach.

All subsequent steps were performed in RStudio 2022.12.0+353 for macOS (RStudio Team 2021) using R v4.2.1 and the following packages: ggplot2 v3.4.1 (Wickham et al. 2019), ggVennDiagram v1.2.0 (Gao, Yu, and Dusa 2022), plyr v1.8.7 (Wickham 2020), tidyverse v1.3.2 (Wickham and RStudio 2019), GO.db v3.15.0 (Carlson 2019), AnnotationHub v3.4.0 (Bioconductor Package Maintainer et al. 2021), KEGGREST v1.36.3 (Tenenbaum, Volkening, and Maintainer 2023) and DESeq2 v1.36.0 (Love, Huber, and Anders 2014).

Steps 2) and 3) were performed separately for the CRG13 and CRG14 data and then merged to keep the overlap of both datasets.

3) Using the gene sets described in 2) and the transcriptome data generated based on the bioprocess runs shown in **Fig. 1A****,** two target gene lists were created for each dataset (CRG13 and CRG14): a) the union list comprising all genes that were present in the 'union' gene set and belonged to the 10% highest expressed genes over all samples, that means throughout the whole bioprocess across all conditions described in **Fig. 1A****.** As this set contained more genes, the expression cut-off was more stringent and b) the intersection list comprising all genes that were present in the intersection gene set and belonged to the 50% highest expressed genes over all samples throughout the bioprocess across conditions described in **Fig. 1A****.** As this set contained fewer genes but with higher confidence of their relevance, the expression cut-off was less stringent.

4) The overlap of targets from both datasets was extracted and the union and the intersection lists were merged into one table. Genes were annotated with information from the UniProt KB.

### Example 1.3: Generation and evaluation of single cell clones with single ECM knock-outs

8 genes (Lpl, Fn1, Bgn, Lamb1, Nid1, Pxdn1, Lgals3bp, Hspg2) identified as ECM or ECM-related proteins were selected as targets for the generation of CHO DG44 single cell clones with single knock-outs. For all ECM genes, gRNAs targeting early exons were designed using Geneious Prime or Benchling software in combination with the annotated genome of an in-house CHO DG44 suspension cell line. To perform knock-outs, RNP complexes were formed by incubating 7.5 pmol CRIPSR nuclease with 7.5 pmol of the respective target gRNA for 15 min. Subsequently, 2E5 CHO DG44 cells were transfected with the complexes using a Neon transfection system (Thermo), transferred into 1 mL CD DG44 medium and incubated at 36.8 °C and 7.5% CO₂. On day 3 after transfection, single clones were isolated using CellCelector nanowell plates (Sartorius) and a CellCelector system (Sartorius). After an incubation period of 4 days (36.8 °C and 7.5% CO₂), the CellCelector system was used to automatically identify outgrown colonies derived from a single cell and to transfer 96 clones to 384 well plates. Cell growth was monitored with a CellMetric imaging system (Solentim) and clones reaching > 17% confluency were transferred to 96 well plates. Following the isolation of genomic DNA using DNA QuickExtract^{™} DNA Extraction Solution 1.0 (Lucigen), PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to generate InDel profiles of the edited clones. For each target, 12 clones showing exclusively out-of-frame InDels and thus a complete functional knock-out of the respective HCP were identified and expanded up to shake flask level. Afterwards, the impact of the single knock-outs on the growth and productivity performance of the edited cell clones under bioprocess conditions was evaluated. For that purpose, 1E6 cells of each knock-out clone as well as unedited CHO DG44 for comparison reasons (in triplicates) were transfected with 10 mg of a proprietary expression plasmid coding for a monoclonal antibody and DHFR as a selective marker. In view of the large number of clones tested, the experiments were conducted in five rounds, wherein CHO DG44 served as reference or control in each experiment.

2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each run and normalized to the respective mean values obtained for the controls (unedited CHO DG44 cells). As summarized in Fig. 2, none of the single knock-outs resulted in a significantly negative impact on growth, process duration, final titer or specific productivity, whereas a positive impact on process duration and final titer was observed in cells containing an Fn1 knock-out.

### Example 1.4: Knock-out of Fn1 in antibody-expressing production clones

In order to confirm the positive impact of an Fn1 knock-out on process duration and final titer, the gene was knocked-out in 4 different CHO DG44 production clones stably expressing a monoclonal antibody and the performance of the resulting knock-out cell lines in a fed-batch bioprocess was analyzed.

For each of the 4 production clones, RNP complexes were formed by incubating 3.75 pmol CRIPSR nuclease with 3.75 pmol of the Fn1 (also called FN / FN1 herein) target gRNA for 15 min. Subsequently, 2E5 CHO DG44 cells were transfected with the complexes using a Neon transfection system (Thermo), transferred into 1 mL proprietary cultivation medium and incubated at 36.8 °C and 7.5% CO₂. 2 days later, the transfected cell pools were transfected a second time with RNP complexes as described above, followed by a third round of transfection after 2 additional days. Following the expansion of the cell pools up to shake flask level, genomic DNA was isolated using DNA QuickExtract^{™} DNA Extraction Solution 1.0 (Lucigen) and PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to determine the proportion of out-of-frame InDels (and therefore the proportion of functional knock-outs) of Fn1 in the cell population. Afterwards, the impact of the Fn1 knock-out on the growth and productivity performance of the edited clones under bioprocess conditions was evaluated. For that purpose, each knock-out clone as well as the respective unedited production clones for comparison reasons were used to run fed-batch bioprocesses in 125 mL shake flask in triplicates: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each run.

As summarized in **Fig. 1G** to **Fig. 1J****,** the Fn1 knock-out resulted increased final titer and process duration compared to the unedited production clones, whereas peak VCC and specific productivity were not significantly different.

Therefore, a Fn1 knock-out, Fn1 also representing a highly abundant and difficult to remove HCP in addition to its nature as ECM -as such identified as likely dispensable for cell integrity based on the methods identified herein - represents an interesting partner for creating multiple knock-outs.

References in Example 1 are: Babraham Bioinformatics. 2010. 'FastQC'. http://www.bioinformatics.babraham.ac.uk/projects/fastqc/. Bioconductor Package Maintainer, Martin Morgan, Marc Carlson, Dan Tenenbaum, Sonali Arora, Valerie Oberchain, Kayla Morrell, and Lori Shepherd. 2021. 'AnnotationHub: Client to Access AnnotationHub Resources'. Bioconductor version: Release (3.14). https://doi.org/10.18129/B9.bioc.AnnotationHub. Bolger, Anthony M., Marc Lohse, and Bjoern Usadel. 2014. 'Trimmomatic: A Flexible Trimmer for Illumina Sequence Data'. Bioinformatics 30 (15): 2114-20. https://doi.org/10.1093/bioinformatics/btu170. Bray, Nicolas L., Harold Pimentel, Páll Melsted, and Lior Pachter. 2016. 'Near-Optimal Probabilistic RNA-Seq Quantification'. Nature Biotechnology 34 (5): 525-27. https://doi.org/10.1038/nbt.3519. Carlson, M. 2019. 'GO.Db: A Set of Annotation Maps Describing the Entire Gene Ontology Assembled Using Data from GO'. http://bioconductor.org/packages/GO.db/. Clerc, Olivier, Madeline Deniaud, Sylvain D Vallet, Alexandra Naba, Alain Rivet, Serge Perez, Nicolas Thierry-Mieg, and Sylvie Ricard-Blum. 2019. 'MatrixDB: Integration of New Data with a Focus on Glycosaminoglycan Interactions'. Nucleic Acids Research 47 (Database issue): D376-81. https://doi.org/10.1093/nar/gky1 035. Ewels, Philip, Måns Magnusson, Sverker Lundin, and Max Käller. 2016. 'MultiQC: Summarize Analysis Results for Multiple Tools and Samples in a Single Report'. Bioinformatics 32 (19): 3047-48. https://doi.org/10.1093/bioinformatics/btw354. Gao, Chun-Hui, Guangchuang Yu, and Adrian Dusa. 2022. 'GgVennDiagram: A "ggplot2" Implement of Venn Diagram'. https://CRAN.R-project.org/package=ggVennDiagram. Love, Michael I., Wolfgang Huber, and Simon Anders. 2014. 'Moderated Estimation of Fold Change and Dispersion for RNA-Seq Data with DESeq2'. Genome Biology 15 (12): 550. https://doi.org/10.1186/s13059-014-0550-8. RStudio Team. 2021. 'RStudio: Integrated Development Environment for R'. Boston, MA: RStudio, PBC. http://www.rstudio.com/. Tenenbaum, Dan, Jeremy Volkening, and Bioconductor Package Maintainer. 2023. 'KEGGREST: Client-Side REST Access to the Kyoto Encyclopedia of Genes and Genomes (KEGG)'. Bioconductor version: Release (3.16). https://doi.org/10.18129/B9.bioc.KEGGREST. The UniProt Consortium. 2021. 'UniProt: The Universal Protein Knowledgebase in 2021'. Nucleic Acids Research 49 (D1): D480-89. https://doi.org/10.1093/nar/gkaa1100. Wickham, Hadley. 2020. `Plyr: Tools for Splitting, Applying and Combining Data'. https://CRAN.R-project.org/package=plyr. Wickham, Hadley, Winston Chang, Lionel Henry, Thomas Lin Pedersen, Kohske Takahashi, Claus Wilke, Kara Woo, Hiroaki Yutani, and RStudio. 2019. 'Ggplot2: Create Elegant Data Visualisations Using the Grammar of Graphics'. https://CRAN.R-project.org/package=ggplot2. Wickham, Hadley and RStudio. 2019. 'Tidyverse: Easily Install and Load the "Tidyverse"'. https://CRAN.R-project.org/package=tidyverse.

### Example 2 Generation of HCP profiles and systematic identification of HCP knock-out targets

In a completely different setting aiming at identifying those HCPs to be knocked-out to obtain a CHO cell with an advantageously reduced HCP content, HCPs were first analyzed in a variety of samples in a systematic manner. To this end, the Uniprot Chinese hamster reference proteome was used as basis for an initial classification to identify the targets of outstanding interest.

HCP profiles were generated for a number of mAb-expressing CHO production clones that were processed under industrial USP (ambr250 fed-batch process) and DSP (standard ProA capture, virus inactivation [VI], cation exchange [CEX] and anion exchange [AEX] polishing steps) conditions. The different production clones, expressed antibodies, applied temperature and pH conditions as well as the sampling regime are shown in **Figure 2E****.**

All samples were analyzed with SWATH LC-MS (for methodology, cf. Krasny et al., Journal of Proteomics Volume 189, 2018, Sim, et al., Sci Data 7, 263,2020) and for each sample the present HCPs were identified and quantified using the Uniprot Chinese hamster reference proteome.

Based on the HCP profiles generated via peptide-based analyses in a complex experimental line-up, two groups of relevant HCP knock-out targets were then identified:
(1) Abundant core HCPs (acHCPs), comprising those HCPs that were detected in all samples taken before or on the harvest day (labelled by the box "abundant HCPs" in **Fig. 2E** and **Table 2**) and that were present at quantifiable amounts (> LLOQ) in all these samples.
(2) Difficult-to-remove HCPs, comprising those HCPs that were detected in at least one of the samples taken during the DSP processing (labelled by the box "difficult-to-remove HCPs" in **Fig. 2E** and **Table 3**).

After each standard purification scheme using a cascade of purification steps was applied as detailed in Example 1, a sample was taken after each step and the sample was analyzed by SWATH LC-MS as well.

As a result of this extensive sample testing, a group of abundant core HCPs (cf. middle column in **Fig. 2E**) and of difficult-to-remove HCPs (cf. right column in **Fig. 2E**) was identified. The relevant HCP knock-out targets from the group of abundant core HCPs (= acHCPs) were defined based on the following criteria: (i) the HCPs are present in all samples labeled by the middle column in **Fig. 2E****,** and (ii) the are present at quantifiable amounts (> LLOQ, cf. **Fig. 2B**)**.** This information was then aligned with the UniProt Chinese Hamster reference genome.

Figure 2A to D shows the identification process, classification and quantities of abundant core HCPs: in total, 1,254 different HCPs were detected in samples taken before or on harvest day, but only 351 of them were found in all these samples. 67 out of those were additionally present at precisely quantifiable amounts (> LLOQ) in all samples and were defined as abundant core HCPs (**acHCPs** see **Table 2**). The identified abundant core HCP species were further characterized regarding their subcellular localization and measured average quantities: 39 HCPs were cytoplasmic proteins present at an average concentration of about 230 mg/L corresponding to about 32.6% of the total HCP amount, 23 HCPs were extracellular (average concentration about 163 mg/L, ca. 23.1% of total HCP) and 5 membrane-bound (27 mg/L, 3.8%) proteins, respectively. According to the criteria defined above, 49 proteins were classified as difficult-to-remove HCPs (**drHCPs** see **Table 3**)**.** Yielding an acHCP and a drHCP list was thus the result of a targeted screening process using bioinformatics and high-throughput analysis steps of a huge number of exemplary samples with the idea to obtain a guidance on relevant HCPs of major interest to be knocked-out. After analyzing (wet lab and in silico) a pool of 1.254 HCPs (present status, further work is ongoing), this object could be achieved by defining acHCPs and drHCPs as "core" HCPs of major importance in view of their abundance and/or inherent removal difficulty. After the initial data analysis characterization, a list of target HCPs of particular interest was identified based on the above screening. Three subsets of HCP targets were defined as follows: first, all relevant HCPs that could be quantified were identified (data not shown here). Next, HCPs were sub-categorized into acHCPs and drHCPs (cf. **Tables 2** and **3**)**.** To complete the initial classification of HCPs as a basis for subsequently identifying targeted knock-out panels, the relevant HCP knock-out targets from the group of difficult-to-remove HCPs were defined based on the following criterion: the HCPs are present in at least one of the samples shown in the right column in **Fig. 2E****,** i.e., the HCP cannot be removed by at least one of the purification strategies used.

### Example 3: Generation and evaluation of single cell clones with single HCP KOs

With the above information on ECM and ECM-related protein expression profiles on the one hand side, and the additional information on HCPs, namely acHCPS and drHCPs in general, a complex matrix of single and multiple knock-out strategies was defined and performed as detailed in the following.

17 genes (Lpl, Fn1, Prdx1 , Bgn, Spp1, Thbs1, Ctsb, Psap, Tnx, Lamb1 , Itih5, Nid1, Pxdn1, Lgals3bp, Pcolce, Timp, Hspg2, cf. **Table 3**) identified as abundant core and/or difficult-to-remove HCPs, and in part also idependently identified as ECM or ECM-related protein HCPs (cf. **Table 1**) were selected as targets for the generation of CHO DG44 single cell clones with single knock-outs. For all HCP genes, gRNAs targeting early exons were designed using Geneious Prime or Benchling software in combination with the annotated genome of an in-house CHO DG44 suspension cell line. To perform knock-outs, RNP complexes were formed by incubating 7.5 pmol CRIPSR nuclease with 7.5 pmol of the respective target gRNA for 15 min. Subsequently, 2E5 CHO DG44 cells were transfected with the complexes using a Neon transfection system (Thermo), transferred into 1 mL CD DG44 medium and incubated at 36.8 °C and 7.5% CO₂. On day 3 after transfection, single clones were isolated using CellCelector nanowell plates (Sartorius) and a CellCelector system (Sartorius). After an incubation period of 4 days (36.8 °C and 7.5% CO₂), the CellCelector system was used to automatically identify outgrown colonies derived from a single cell and to transfer 96 clones to 384 well plates. Cell growth was monitored with a CellMetric imaging system (Solentim) and clones reaching > 17% confluency were transferred to 96 well plates. Following the isolation of genomic DNA using DNA QuickExtract^{™} DNA Extraction Solution 1.0 (Lucigen), PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to generate InDel profiles of the edited clones. For each target, 12 clones showing exclusively out-of-frame InDels and thus a complete functional knock-out of the respective HCP were identified and expanded up to shake flask level. Afterwards, the impact of the single knock-outs on the growth and productivity performance of the edited cell clones under bioprocess conditions was evaluated. For that purpose, 1E6 cells of each knock-out clone as well as unedited CHO DG44 for comparison reasons (in triplicates) were transfected with 10 mg of a proprietary expression plasmid coding for a monoclonal antibody and DHFR as a selective marker. In view of the huge number of clones tested, the experiments were conducted in five rounds each, always using CHO DG44 as control. This five-partite experimental set-up is reflected by the dotted lines in Figure 3A to D.

2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each run and normalized to the respective mean values obtained for the controls (unedited CHO DG44 cells). As summarized in **Figure 3** **A to D,** none of the single knock-outs resulted in a significantly negative impact on growth, process duration, final titer or specific productivity, whereas even a positive impact on process duration and final titer was observed in cells containing an Fn1 knock-out.

### Example 4: Generation and evaluation of cell pools with single HCP knock-outs

10 further genes (Pkm, Cspg4, Ldha, Vim, Hspa5, Ppib, Hsp90aa1, Lama5, Fstl1, Aebp1) identified as abundant core and/or difficult-to-remove HCPs were selected as targets for the generation of CHO DG44 cell pools with single knock-outs.

Further experiments with HCPs qualifying or additionally qualifying as ECM and ECM-related proteins planned in a comparable way are ongoing.

For all HCP genes, gRNAs targeting early exons were designed using Geneious Prime or Benchling software in combination with the annotated genome of an in-house CHO DG44 suspension cell line. To perform knock-outs, RNP complexes were formed by incubating 3.75 pmol CRIPSR nuclease with 3.75 pmol of the respective target gRNA for 15 min. Subsequently, 2E5 CHO DG44 cells were transfected with the complexes using a Neon transfection system (Thermo), transferred into 1 mL CD DG44 medium and incubated at 36.8 °C and 7.5% CO₂. 2 days later, the transfected cell pools were transfected a second time with RNP complexes as described above, followed by a third round of transfection after 2 additional days. Following the expansion of the cell pools up to shake flask level, genomic DNA was isolated using DNA QuickExtract^{™} DNA Extraction Solution 1.0 (Lucigen) and PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to determine the proportion of out-of-frame InDels (and therefore the proportion of functional knock-outs) of the respective HCP in the cell population. Afterwards, the impact of the single knock-outs on the growth and productivity performance of the edited cell pools under bioprocess conditions was evaluated. For that purpose, 1E6 cells of each knock-out pool as well as unedited CHO DG44 for comparison reasons were transfected with 10 mg of a proprietary expression plasmid coding for a monoclonal antibody of interest and DHFR as a selective marker (all transfections were done in triplicates). 2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%.

As key performance indicators highest viable cell concentration (=peak VCC), process duration, final titer and mean specific productivity were determined for each run. As summarized in Fig. 4A to D, none of the single knock-outs resulted in a pronounced negative impact on growth, process duration, final titer or specific productivity.

### Example 5: Generation and evaluation of single cell clones with multiple HCP KOs

7 knock-out targets (Bgn, Fn1, Lpl, Nid1, Pcolce, Pxdn, Thbs) were selected based on the data generated in **Example 3** for the generation of CHO DG44 single cell clones with multiple knock-outs. Additionally, five of the targets (Bgn, Fn1, Lpl, Nid1 and Pxdn) were specifically attractive as these were independently characterized as ECM and ECM-related proteins.

For each of the 7 targets RNP complexes were formed by incubating 1.1 pmol CRIPSR nuclease with 1.1 pmol of the respective target gRNA for 15 min, using the same gRNAs as described in **Example 3.** Subsequently, all 7 RNP reactions were pooled together, 2E5 CHO DG44 cells were transfected with the RNP pool using a Neon transfection system (Thermo), transferred into 1 mL CD DG44 medium and incubated at 36.8 °C and 7.5% CO₂. 2 days later, the transfected cell pool was transfected a second time with pooled RNP complexes as described above, followed by a third round of transfection after 2 additional days. 3 days after the third round of transfection, single clones were isolated using CellCelector nanowell plates (Sartorius) and a CellCelector system (Sartorius). After an incubation period of 4 days (36.8 °C and 7.5% CO₂), the CellCelector system was used to automatically identify outgrown colonies derived from a single cell and to transfer 172 clones to 384 well plates. Cell growth was monitored with a CellMetric imaging system (Solentim) and 158 clones reaching > 17% confluency were transferred to 96 well plates. Following the isolation of genomic DNA using DNA QuickExtract^{™} DNA Extraction Solution 1.0 (Lucigen), PCR reactions were performed with appropriate primer pairs resulting in 300-500 bp amplicons covering the target regions around the cut sites. The obtained DNA fragments were subjected to Sanger sequencing (Microsynth Seqlab) and analyzed with the open-source software Inference of CRISPR edits (ICE) v3.0 (Synthego) to generate InDel profiles of the edited clones. All clones showing exclusively out-of-frame InDels and thus complete functional knock-outs for at least 4 out of the 7 addressed HCP targets were identified and expanded up to shake flask level. The observed knock-out combinations and the number of clones which were found for each of them are summarized in Fig. 5. Afterwards, the impact of the different knock-out combinations on growth and productivity performance of the edited cell clones under bioprocess conditions was evaluated. For that purpose, 1E6 cells of each knock-out clone as well as unedited CHO DG44 for comparison reasons (in quadruplicate) were transfected with 10 mg of a proprietary expression plasmid coding for a monoclonal antibody and DHFR as a selective marker. 2 days post transfection the cells were transferred to a proprietary selective medium, cultivated at 36.8 °C, 7.5% CO₂ and linear shaking at 110 rpm and subcultured every 3-4 days. About 10-14 days after transfection, when increasing cell concentrations and viabilities indicated full recovery from selective pressure, the cultures were used to run fed-batch bioprocesses in 125 mL shake flask: 25 mL proprietary production medium were inoculated with 3E5 cells/mL and cultured at 36.8 °C, 7.5% CO₂ and linear shaking at 110 rpm until the end of the run. Starting on day 3 after inoculation, every day defined amounts of proprietary feed medium A and B as well as glucose were added according to an in-house feeding regime. Viable cell concentration and viability, glucose and lactate concentration as well as antibody titers were measured at defined days of the process using a Vi-Cell cell counter (Beckman Coulter), a Biosen C-Line device (EKF) and an Octet device (Sartorius), respectively. A run was ended once the viability dropped below 70%. As key performance indicators highest viable cell concentration (=peak VCC), process duration, final titer and mean specific productivity were determined for each.

As summarized in **Fig. 6****,** for none of the analyzed HCP knock-out combinations a pronounced negative impact on growth, process duration, final titer or specific productivity compared to unedited CHO DG44 cells was observed. This confirms that the targets represent suitable targets for multiple KOs for reducing the overall HCP load, whilst at the same time maintaining the cellular integrity.

In a further test series, the following panel of proteins was combined (at least two, or more than two knock-outs): Lgals1, Adamts1, Adamts7, Col5a2, Col7a1, Efemp2, Lamc1, Ltbp3, Mfge8, Mmp28, Sparc, Tgfb1, Tgfb3, Tgm2, and Wnt4. This is due to the fact that these targets turned out to be highly promising in view of their extracellular localization represented suitable candidates, as the cells are not needed for adhesion. Individual knock-outs yielded highly promising results. Combination testing is currently ongoing.

In yet a further interesting panel, Fn1, Hspg2, Pxdn and Lamb1 were combined. In view of their outstanding individual capacity to reduce the metabolic burden as well as the HCP total load whilst maintaining or even improving the bioprocess performance, a quadruple knock-out is presently tested - alone or as basis for further combinations.

As the quadruple setting initially Fn1, Hspg2, Pxdn and Lamb1 as knock-out targets yielded very promising first results, a further expanded multiplexing knock-out panel comprising Lpl, Nid1 (different isoforms), Pxdn, Bgn, Lgals3bp and Lamb1 is presently being tested. Again, this 6x KO will serve as basic platform for combinations with further ECM/ECM-related protein KO targets to stepwise optimize the CHO cell regarding its ECM profile.

### Example 6: Nuclease Testing

Having confirmed that multiple HCP knock-outs can be inserted into a CHO-target cell of interest simultaneously without a negative impact on the overall viability and production capability of the target cell, several nucleases were chosen for testing to define the optimum set-up for a multiplex knock-out experiment. To this end, several multiple targets (at least 5 at the same time) as shown in Table 1 were selected. Further, suitable nucleases were provided and the guide RNAs were adapted accordingly to fit the needs of the nuclease of interest and to recognize the respective target sequences, respectively.

It could be shown that certain nucleases perform superior in multiplexing knock-out experiments. Generally, it can be preferable to knock-out more than one HCP target in once experiment to reduce the overall process time needed, the decrease the cellular stress on the target cell and to better control and check the knock-out efficiency and effectiveness.

As it could be demonstrated that several nucleases are suitable for creating multiple desired knock-outs simultaneously, further experiments are currently ongoing to defined alternative strategies.

### Example 7: Further Compositions and Equipment

### Media, Compositions, Kits and Chemicals

For CHO cell cultivation, commercially available media from Thermo Fisher Scientific and Sartorius Stedim Cellca were used.

For further material, 125mL shake flasks of Corning, 24-well Nanowell plates of ALS, 384-well flat clear bottom black microplates of Corning, 96-well PCR plate, non-skirted Brand CASY cups of OMNI Life Science, CASY ton of OMNI Life Science, Enhanced Qsol Cartridge Kit for iQue^{®} screener of Sartorius, iQue^{®} Marker (B/Yellow) of Sartorius, iQue^{®} Validation Beads of Sartorius, NEON^{™} Transfection System 10 µl/100 µl kit of Thermo Fisher Scientific, NucleoSpin^{®} miRNA of Macherey-Nagel, Nunc cell culture platesof Thermo Fisher Scientific, Phusion^{®} High-Fidelity DNA Polymerase of New England Biolabs, pmaxGFP^{®} Vector of Lonza, and QuickExtract DNA Extraction Solutionof Lucigen were usually used, but alternative materials can be used as well. For devices and equipment, the following were used, but alternative equipment as available to the skilled person can of course be used as well: Cell counter of Beckman Coulter, Cell counter of Roche, Cell imager of Solentim, CO2 incubator of Thermo Fisher Scientific, Flow cytometer of Sartorius, Glucose/Lactate Analyzer of EKF-diagnostics, Protein Quantification System of Sartorius, Real-Time PCR device of Thermo Fisher Scientific, Single cell cloning device of Automated Lab Solutions, and a Transfection device of Thermo Fisher Scientific.

### Example 8: sgRNAs

To knock out the desired gene targets, sgRNAs were designed targeting an early exon present in all available transcript variants utilizing the bioinformatics tool Geneious Prime 11.0.11 (Biomatters, Auckland, New Zealand) or the cloud-based CRISPR Guide RNA Design Software (https://www.benchling.com). The sgRNAs were then ordered and synthesized by different manufacturers, depending on the nuclease of interest the sgRNA was designed for. To generate frameshift mutations in the desired HCP genes, usually at least three sgRNAs for each target were evaluated for their KO-efficiency regarding their ability to generate desirable InDels leading to a functional KO. Desirable InDels are defined as InDels which are not multiples of three, show a large prevalence (>30%) in the heterogenous sequence pool and lead to early stop-codons in translated protein sequences. In the first KO-round, the CHO DG44 host cell pool was transfected with sgRNAs for each target gene as shown in Tables 1 to 3 above. R-buffer transfected cells served as negative controls and pMaxGFP as positive control for the determination of transfection efficiency and assessment of the viability of the transfection setup of devices and reagents. Transfection efficiency was determined in two separate LPs 24 h after transfection in three technical replicates, gating and efficiencies (data not shown here). The total mean of transfection efficiency was measured to be 96.9% (data not shown). Genomic DNA was extracted 48 h after transfection. Via Sanger sequencing, InDel events (i.e. insertions or deletions) at cut sites of the respective site-directed endonuclease were assessed in DNA fragments of pools and clones previously amplified via PCR. The chromatograms from sequencing reactions were viewed using Geneious Prime 11.0.11 (Biomatters, Auckland, New Zealand). Also, a quality score was assigned to each sequencing reaction corresponding to the percentage of untrimmed bases that are high quality, where high quality is defined as a function of the percentage of ambiguities present in the sanger traces. The results obtained via Sanger sequencing could e.g. be used for assessing specific editing efficiencies and InDel profiles. Selected gRNAs used (only crRNA, 5' to 3' w/o PAM) are shown with SEQ ID NOs: 129 to 153.

Wild type CHO cells and all derived clones were handled under sterile conditions at all times. CHO cell cultivation was always performed at 36.8 °C, under 7.5% CO₂ atmosphere while shaking at 110 rpm. Only cultures in plates (i.e. nanowell plates, 384-well plates, 96-well plates, 24-well plates, and 12-well plates) were incubated without shaking. Culture parameters like the viable cell concentration (VCC), total cell concentration (TCC), viability and peak diameter were determined using the CASY cell counter. From the pools transfected with a site-directed endonuclease of interest, stable single-cell clones were generated in order to be assessed in fed-batch mode.

Single-cell cloning was usually performed 3 days after transfection with the respective site-directed endonuclease.

After genetic screening, knock-out clones (KO-clones) were resuspended and diluted and clones were further incubated and cell growth was consistently monitored by measuring confluency.

KO-clones transfected with the respective expression vector (as described above) were expanded. Once good cell growth (about 8 to 10 * 10⁵ cells/mL after days) and a good cell viability of > 90% could be restored after the transfection procedure, a fed-batch bioprocess was performed for evaluation of culture performance of the respective KO-clones.

Antibody titers of samples obtained from fed-batch cultures were determined using Octet^{®} QKe (Sartorius) system according to the manufacturer's instructions. Titers were determined by thawing (RT) supernatant samples collected during fed-batch cultivation. The results were then analysed using the Forte Bio Data analysis 9.0 software (Sartorius Lab Instruments GmbH & Co. KG, Goettingen, Germany).

To screen HCPs present in supernatants from harvested samples, amino acid analysis and peptide fragment matching against protein databases to designate present HCPs with UniProt Identifiers through external liquid chromatography (LC-MS) (Alphalyse A/S, 5230 Odense M, Denmark) was performed. Additionally, for HCP profiling screens, SWATH LC-MS was used (cf. Krasny et al., Journal of Proteomics Volume 189, 2018).

The relative abundance of each HCP could then be calculated utilizing the production titer provided by Sartorius. Further data about the cellular localization, amino acid sequence, gene ID and gene structure as well as functions of each HCP was acquired from the UniProt Database. The genetic sequences of all proteins were acquired through NCBI and verified.

## Claims

1. A CHO cell with a modified extracellular matrix (ECM) profile, **characterized by** a reduced load of at least one endogenous protein being an ECM protein or an ECM-related protein.

2. The CHO cell of claim 1, wherein said CHO cell comprises:
at least one modification in at least one endogenous coding sequence coding for at least one endogenous ECM protein or endogenous ECM-related protein selected from the group summarized in Table 1, or of any homologue, orthologue, or paralogue thereof.

3. The CHO cell of claim 1 or 2, wherein said CHO cell comprises at least two or more modifications in two or more coding sequences coding for two or more endogenous ECM proteins and/or endogenous ECM-related proteins, wherein the at least one further modification of the at least two or more modifications is a modification of a sequence encoding an ECM protein or an ECM-related protein selected from the group summarized in Table 1, Table 4, Table 5, or Table 6 or any homologue, orthologue, or paralogue thereof, preferably wherein the at least one further modification of the at least two or more modifications is a modification of a sequence encoding an ECM protein or an ECM-related protein having an amino acid sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, respectively.

4. The CHO cell according to any of the previous claims, wherein said CHO cell comprises at least two or more modifications in two or more coding sequences coding for two or more endogenous ECM proteins and/or endogenous ECM-related proteins, wherein at least one of the at least two or more modifications is in a sequence encoding an ECM protein or an ECM-related protein being selected from the group consisting of Fibronectin, Basement membrane-specific heparan sulfate proteoglycan core protein, Peroxidasin, Biglycan, Galectin-3 binding protein, Laminin subunit beta-1, Calreticulin, Galectin, Glypican, or any homologue, orthologue, or paralogue thereof; more preferably wherein at least one of the at least two or more modifications is in a sequence encoding an ECM protein or an ECM-related protein having an amino acid sequence corresponding to SEQ ID NO: 2, 12, 29, 36, 38, 54, 55, 77, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: SEQ ID NO: 2, 12, 29, 36, 38, 54, 55, 77, respectively.

5. The CHO cell according to any of the previous claims, wherein the at least one further modification is a modification of a sequence encoding an ECM protein or an ECM-related protein selected from the group summarized in Table 1, Table 4, Table 5, or Table 6, or any homologue, orthologue, or paralogue thereof and/or wherein the at least one further modification is a modification of a sequence encoding an abundant core HCP (acHCP) and/or a difficult to remove HCP (drHCP) selected from the group summarized in Table 2 and Table 3 respectively, or any homologue, orthologue, or paralogue thereof.

6. The CHO cell according to any of the previous claims, wherein said at least one modification leads to the reduced transcription and/or reduced functional expression of said endogenous ECM protein(s) and/or ECM-related protein(s) thereby lowering the total content of endogenous ECM proteins and/or endogenous ECM-related proteins.

7. The CHO cell according to any of the previous claims, wherein said CHO cell comprises three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, ortwenty or more modifications in three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, or twenty or more coding sequences, respectively, wherein each of said modifications affects a protein being classified as an endogenous ECM protein or an endogenous ECM-related protein, preferably wherein at least one ECM protein or an endogenous ECM-related protein is independently selected from SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 2, 12, 14, 24, 28, 29, 36, 38, 42, 54, 55, 77, 94, 95, 96, 98, 101, 104, 106, 109, 110, 111, 112, 113, 124, 127 or 156, respectively.

8. The CHO cell according to any of the previous claims, wherein said CHO comprises at least one recombinant gene encoding at least one recombinant protein of interest and/or encoding at least one recombinant RNA molecule of interest, preferably wherein said at least one recombinant protein of interest is a therapeutic molecule.

9. The CHO cell according to any of the previous claims, wherein said at least one modification is selected from the group consisting of at least one insertion, at least one deletions, and at least one substitution, including a base edit, or any combination thereof,
preferably wherein said at least one modification is present in exon 1 of the respective endogenous coding sequence
and/or
wherein said at least one modification in said coding sequence is a frame-shift mutation or a point mutation, the point mutation resulting in a stop codon.

10. The CHO cell according to any of the previous claims, wherein said at least one modification in said at least one endogenous coding sequence allows for an improved bioprocess performance, wherein said improved bioprocess performance is **characterized by** an increased concentration of viable cells and/or an increased specific productivity and/or an increased final titer and/or an increased process duration in comparison to a wild-type cell not carrying the at least one modification in its genome.

11. The CHO cell according to any of the previous claims, wherein said at least one modification in said endogenous coding sequence is a frame-shift mutation or a point mutation, the point mutation resulting in a stop codon.

12. The CHO cell according to any of the previous claims, wherein all alleles of said at least one endogenous coding sequence comprise at least one or more of said modification(s).

13. A method for the production of at least one modified CHO cell according to any of the claims 1 to 12 comprising the steps:
(i) providing at least one CHO cell comprising at least one endogenous target nucleic acid segment;
(ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same;
(iii) introducing into said at least one CHO cell the at least one genome editing agent of step (ii);
(iv) obtaining at least one modified CHO cell comprising at least one modification in said at least one endogenous target nucleic acid segment according to step (i);
(v) optionally: selecting a further target nucleic acid segment and repeating steps (i) to (iv) at least one time to obtain at least one modified CHO cell comprising at least one further modification in a further endogenous target nucleic acid segment.

14. A method for the production of at least one recombinant molecule, preferably at least one recombinant protein, of interest comprising the steps:
(a) providing at least one modified CHO cell of claim 13, wherein the at least one modified CHO cell comprises at least one recombinant gene encoding at least one recombinant protein, DNA or RNA of interest;
(b) culturing said at least one target cell in a culture medium such that at least one recombinant molecule of interest is transcribed and/or translated;
(c) harvesting said at least one recombinant molecule of interest;
(d) purifying and/or decontaminating said at least one recombinant molecule, preferably the at least one recombinant protein of interest.

15. A use of at least one modified CHO cell according to any of the claims 1 to 12 for the production of at least one pharmaceutical drug.

16. A kit comprising
(a) at least one modified CHO cell according to any of claims 1 to 12, the CHO cell or the kit comprising:
(b.i) at least one nucleic acid molecule suitable for expressing at least one recombinant molecule of interest, preferably at least one recombinant protein of interest; optionally means for introducing the same into the CHO cell genome, or alternatively
(b.ii) at least one nucleic acid molecule encoding at least one recombinant molecule of interest, preferably at least one recombinant protein of interest; optionally means for introducing the same into the CHO cell genome; or alternatively
(b.iii) at least one nucleic acid molecule suitable for transcribing at least one recombinant RNA molecule of interest; optionally means for introducing the same into the CHO cell genome; or alternatively
(b.iv) at least one nucleic acid molecule encoding at least one recombinant RNA molecule of interest; optionally means for introducing the same into the CHO cell genome; and optionally
(c) culture medium suitable for the at least one modified CHO cell according to step (a)
(i) to replicate the at least one nucleic acid molecule according to any of the steps (b.i), (b.ii), (b.iii), and (b.iv) endogenously or exogenously; and
(ii.a) to express the at least one recombinant protein of interest according to step (b.i) and/or (b.ii), and/or to transcribe the at least one recombinant RNA molecule of interest according to step (b.iii) and/or (b.iv).
